# EUROPEAN PATENT SPECIFICATION

(11) **EP 0 941 034 B1**
(45) Date of publication and mention of the grant of the patent: **26.05.2004**
(21) Application number: 98951487.2
(22) Date of filing: 01.10.1998
(51) Int. Cl.: A61B 17/11

(54) **TRANSANAL ANASTOMOSIS RING INSERTION DEVICE**
EINFÜHRVORRICHTUNG FÜR EINEN TRANSANAL ANASTOMOSE RING
DISPOSITIF D'INTRODUCTION D'UNE BAGUE D'ANASTOMOSE PAR LA VOIE ANALE

(30) Priority: 02.10.1997 US 60859 P
(43) Date of publication of application: 15.09.1999
(73) Proprietor: Sherwood Services AG, 8201 Schaffhausen (CH)
(72) Inventor: PHILLIPS, Paul, Joseph, Middlebury, CT 06762 (US); CORBETT, Robert, Kelly, New Milford, CT 06776 (US); CONNERS, John, A., Fairfield, CT 06430 (US)
(74) Representative: HOFFMANN - EITLE
(86) International application number: PCT/EP1998/006265
(87) International publication number: WO 1999/017662

(56) References cited:
- EP-A- 0 568 774
- EP-A- 0 671 149
- WO-A-81/00668
- US-A- 5 282 810

## Description

### BACKGROUND OF THE INVENTION

### 1. Field of the Invention

The present invention generally relates to a surgical device, and more particularly to an anastomosis ring insertion device for use in anastomosing tubular body organs in connection with, for example, intestinal surgery.

### 2. Prior Art

EP-0,568,774 discloses an anastomosis device which utilises a collet as part of the release mechanism.

Atter a surgical procedure, such as cutting and removing a diseased or cancerous portion of the bowel, the severed ends of the bowel must be anastomosed or rejoined. Several procedures are available for anastomosing two sections of hollow tubular body organs, such as the intestines. Known procedures for anastomosing two sections of hollow tubular body organs together include suturing, stapling or clamping the severed ends together. For example, U.S. Patent Nos. 4,576,167, No. 4,603,693 and No. 4,646,745 are directed to anastomosis procedures utilizing circular surgical staplers for anastomosing two hollow body organs.

Another procedure uses anastomosis buttons and clamps to join two sections of hollow body organs together. U.S. Patent Nos. 3,771,526, No. 4,055,186 and No. 4,154,241 are exemplary of such anastomosis devices. The devices disclosed in these patents utilize inserter rods which are forced upwardly into the rectum through the anus in order to position one half of a clamp device in the lower colon and engage the other half of the clamp device positioned in the upper colon, so as to draw and join the two hollow body organ halves together.

Still another procedure involves use of an anastomotic device that is the subject of U.S. Patent Nos. 4,467,804, No. 4,467,804 and No. 4,766,898, which are assigned to the assignee of the present invention, and marketed and sold under the VALTRAC® trademark. The anastomotic device disclosed under these patents receives the open ends of intestines to be purse stringed and anastomosed over and between a pair of ring members that comprise the VALTRAC®. The ring members have mechanical connecting means which mate with each other in order to produce secure serosa-to-serosa apposition of the ends of the instestines and maintains a satisfactory patency unitl healing occurs and the ring members degrade into small harmless fragments which are eliminated from the body. The ring members, also referred to collectively as a Biofragmentable Anastomosis Ring (BAR), are formed of a bio-absorbable material that permits disintegration of the BAR in a relatively short period of time after healing of the intestines begins, thereby eliminating the need for metal staples and clamps which do not disintegrate and pass through the patient's system. Acceptable materials for manufacturing the BAR are disclosed in U.S. Patent No. 3,297,033 and include, but are not limited to, poly-hydroxy acetic ester and lactate copolymers which are herein incorporated by reference. However, use of this type of anastomotic device requires that the surgeon work solely through an abdominal opening made in the patient, which is disadvantageous when performing anastomosis to the lower bowel due to the limited space available in the pelvic area for performing such a procedure.

U.S. Patent No. 5,464,415 discloses a sutureless anastomosis gun for a BAR for use in sutureless anastomosis of the intestine near the anus. The anastomosis device disclosed in this patent drives an inner sleeve and a ring seat by means of nut and screw rod arrangement such that the end of the rectum is pushed precisely to enter the gap of the BAR and then the BAR is exactly closed about the portion of the intestines to be anastomosed. Thereafter, the ring seat and the BAR are disengaged and the anastomosis gun removed from the rectum. However, the anastomosis gun of this type does not transanally insert one member of the BAR so that an exact closing about the portion of the intestines to be anastomosed is not required.

U.S. Patent Nos. 4,667,673 and 5,282,810, also assigned to the assignee of the present invention, disclose an anastomosis applicator device for mounting the BAR and a method for inserting the applicator and BAR transanally. The device includes a mounting extension for mounting the two halves of the BAR and an inserter which is curved and adapted for transanal insertion. The inserter portion of the applicator passes through the interior of the rectum and out through the exterior of the anus so that the placement of the BAR can be done without the difficulty present in having to access the lower bowel area solely through the pelvic area.

However, it is desirable that further improvements to the transanal applicator be made related to better mounting and application of the device during an anastomosis procedure.

### OBJECTS AND SUMMARY OF THE INVENTION

The invention is defined in claim 1 below, in which the pre-characterizing part corresponds to the disclosure of Applicant's earlier US-A.5,282,810, mentioned above. The dependent claims are directed to optional or preferred features of the subject inventive concept.

A principal object of the present invention is to provide an improved applicator insertion device for surgically inserting a BAR, more particularly an insertion device for surgically inserting a unitary member of a BAR in an open end of the lower bowel through the anal orifice, or through an abdominal insertion. Indeed, the present invention provides an insertion device that swiftly and easily mounts, closes, and releases both unitary members of the BAR in order to clamp the open ends of the bowel in a contiguous manner.

These and other objects of the present invention are realized in a presently preferred embodiment thereof, described by way of example and necessarily by way of limitation, which provides for an insertion device for transanally inserting one of two unitary members of a BAR through the rectum and securing it to the open end of the lower bowel, while inserting the other unitary member through an abdominal opening and securing it to the open end of the upper bowel. The insertion device of the present invention comprises a curved shaft with a handle and knob at the proximal end, a nest formed at the distal end thereof adapted to mount a BAR carrier assembly, and communicating first and second passageways formed through the shaft which terminate at a distal end opening formed through the nest.

The BAR carrier assembly includes unitary male and female members that collectively comprise the BAR, male and female carriers for temporarily mounting both unitary members of the BAR onto the insertion device, a protective cover for safe and comfortable transanal insertion of the insertion device, and a collet/drawbar arrangement for locking in and releasing the BAR from the BAR Carrier Assembly once both unitary members are secured to respective open ends of the upper and lower bowel and then mated together prior to release. In the preferred embodiment, both unitary male and female members include a plurality of openings interposed between scallops in juxtaposition relationship which are formed through the dome of each unitary member. The openings promote the fragmentation of the BAR after a period of time by allowing the BAR to break down into smaller pieces when it begins to disintegrate, thereby allowing the fragments to more easily pass out the body through the stool. The scallops which form the outer edge of each unitary member form a continuous bumpy pattern thereabout that serves to better clamp the open ends of the bowel between the two unitary members of the BAR when mated together, thereby promoting vascularity of healthy bowel tissue during anastomosis.

The knob positioned at the proximal end of the shaft communicates with a threaded body that includes a threaded channel adapted to engage a rod member connected to a flexible shaft member. Formed or attached thereto at the distal end of the shaft member is a drawbar adapted to engage the connectors of the collet. The collet has a body having a distal end that includes an elongated stem with a wedge formed at the free end thereof while a plurality of connectors at the opposite end of the collet body extend generally axially therefrom.

Both male and female carriers have hollow generally tubular bodies with opposed proximal and distal openings, respectively. The proximal opening of the male carrier includes a concentric pattern of connectors adapted for attachment to the distal opening of the female carrier while the distal opening of the male carrier forms a plurality of generally axially extending legs with retaining jaws formed at the free end thereof.

During manufacturing, the female carrier and female member are pre-loaded to the distal end of the insertion device by engaging the female carrier to the shaft and inserting the female carrier through the aperture formed through the dome of the female member. Mating prongs formed on the female member then engage slots formed on the female carrier, thereby seating the female carrier securely in the nest of the insertion device. Further, the collet is pre-loaded to the male carrier by inserting the collet through the male carrier so that the wedge of the collet is placed between the plurality of legs, thereby biasing apart the legs. Prior to inserting the collet through the male carrier, the male member is pre-loaded to the distal end of the male carrier so that the retaining jaw formed at the free end of each leg of the carrier is advanced through the aperture formed in the dome of male member. Once the legs are advanced through the aperture of the male member so that the retaining jaws engage the peripheral area around the aperture, the wedge of the collet is then inserted through the male carrier until the bump formed opposite of each retaining jaw engages a retaining ridge formed around the circumference of the wedge and biases apart the plurality of legs. A biasing means, for example a garter spring, is received around the inside of each retaining jaw so as to provide a counter force that locks the wedge of the collet between the legs of the male carrier, thereby locking the male member to the distal end of the male carrier.

In operation, a surgeon makes an abdominal opening through an incision made in the patient in order to access the bowel area. The surgeon then severs the bowel into an upper bowel section and a lower bowel section and then cleans the edges of each respective bowel section of any cancerous or diseased portion in order to ensure healthy tissue is present for anastomosis. The pre-loaded male member with the male carrier attached thereto is then inserted through the abdominal opening by the surgeon and the edge of the upper bowel section is secured around the outer edge of the male member in a contiguous manner thereto by employing, for example a purse string stitch pattern, using a absorbable suture material to secure the edge of the upper bowel section. After the upper bowel section is secured, the surgeon inserts the distal end of the shaft, with the pre-loaded female member attached thereto, through the rectum of the patient and into the bowel until the shaft appears through the lumen of the lower bowel section.

Once the shaft has been inserted through the lower bowel, the edge of the lower bowel section is secured around the outer edge of the female member in a manner similar to the securement of the upper bowel section to the male member. Upon securing both upper and lower bowel sections, the surgeon attaches the male carrier with its connected male member to the distal end of the female carrier, so as to place the BAR Carrier Assembly in the open position with the female member spaced apart from the male member along the axis of the Carrier Assembly. Further, when the male carrier is attached to the female carrier, the collet engages the drawbar so that the collet can release the male member at the appropriate time when the device is actuated.

Once the BAR Carrier Assembly is in the open position, the surgeon actuates the device in order to place the Carrier Assembly in the closed position and mate the male member to the female member. In order to actuate the device and place the BAR Carrier Assembly in the closed position, the surgeon rotates the knob of the device between the thumb and forefinger in a clockwise direction, which causes the male and female carriers to be pulled into the second passageway of the shaft due to the drawbar's engagement with the collet. As both carriers are pulled into the second passageway by the drawbar, the male member is brought to mating engagement with the female member seated in the nest of the shaft, thereby placing the BAR Carrier Assembly in the closed position.

During the mating sequence between the male member and the female member, the mating prongs of the female member engage respective slots formed in the tubular member of the male member in such a manner as to maintain contiguous contact between the opposing portions of the upper and lower bowel sections secured to the outer edge of each respective unitary member. Once the two unitary members are mated and both sections of the bowel secured, the surgeon places the BAR Carrier Assembly in the release position by rotating the knob in a counter-clockwise direction. This counter-clockwise rotation causes the drawbar to move in an axial direction away from the knob, thereby causing the collet to move forward relative to the male carrier. The relative forward movement of the collet also removes the wedge from between the plurality of legs of the male carrier and releases the mated unitary members of the BAR from the BAR Carrier Assembly. Once release of the BAR has been effected, the surgeon withdraws the shaft of the device from the lower bowel section of the patient and the procedure is completed.

In an alternative embodiment, the proximal end of the collet has a cross bar-like configuration similar to the one formed at the distal end of the drawbar in the preferred embodiment, while the alternative drawbar has a plurality of connectors similar to those formed at the proximal end of the collet body in the preferred embodiment. The alternative embodiment, with the exception of the collet and drawbar, is structurally and operatively the same as the preferred embodiment.

### BRIEF DESCRIPTION OF THE DRAWINGS

FIG. **1** is a perspective view of the anastomotic device according to the present invention;
FIG. **2** is a partial cross-section view of the shaft and handle of the anastomotic device shown in FIG. **1** according to the present invention;
FIG. **3** is an exploded view of the distal end of the anastomotic device illustrating the various components of the BAR Carrier Assembly according to the present invention;
FIG. **4** is a side view of the male member according to the present invention;
FIG. **5** is a cross-section view of the male member shown in FIG. **4** along lines **A-A** according to the present invention;
FIG. **6** is a perspective view of the male member shown in FIG. **4** according to the present invention;
FIG**. 7** is a top view of the male member shown in FIG. **4** according to the present invention;
FIG. **8** is a side view of the female member according to the present invention;
FIG. **9** is a cross-section view of the female member shown in FIG. **8** along lines **B-B** according to the present invention;
FIG. **10** is a top view of female member shown in FIG. **8** according to the present invention;
FIG. **11** is a perspective view of the female member shown in FIG. **8** according to the present invention;
FIG. **12** is a side view of the male carrier according to the present invention;
FIG. **13** is a cross-section view of the male carrier shown in FIG. **12** along lines **C-C** according to the present invention;
FIG. **14** is a perspective view of the male carrier shown in FIG. **12** according to the present invention;
FIG. **15** is an end view of the male carrier shown in FIG. **12** according to the present invention;
FIG. **16** is a side view of the collet according to the present invention;
FIG. **17** is a cross-section view of the collet shown in FIG. **16** along lines **D-D** according to the present invention;
FIG. **18** is a perspective view of the collet shown in FIG. **16** according to the present invention;
FIG. **19** is a side view of the female carrier according to the present invention;
FIG. **20** is a cross-section view of the female carrier shown in FIG. **19** along lines **E-E** according to the present invention;
FIG. **21** is a perspective view of the female carrier shown in FIG. **19** according to the present invention;
FIG. **22** is an end view of the female carrier shown in FIG. **19** according to the present invention;
FIG. **23** is a side view of the distal end of the shaft according to the present invention;
FIG. **24** is a cross-section view of the distal end of the shaft shown in FIG. **23** along lines **F-F** showing the nest and second passageway according to the present invention;
FIG. **25** is an end view of the distal end of the shaft shown in FIG. **23** according to the present invention;
FIG. **26** is a perspective view of the drawbar according to the present invention;
FIG. **27** is an end view of the drawbar shown in FIG. **26** according to the present invention;
FIG. **28** is a perspective view of the distal end of the shaft illustrating the BAR Carrier Assembly in the open position;
FIG. **29** is a cross-section view of the distal end of the shaft and BAR Carrier Assembly shown in FIG. **28** along lines **G-G** according to the present invention;
FIG. **30** is a perspective view of the distal end of the shaft illustrating the BAR Carrier Assembly in the closed position;
FIG. **31** is a cross-section view of the distal end of the shaft and BAR Carrier Assembly shown in FIG. **30** along lines **I-I** according to the present invention;
FIGS. **31A-31C** illustrate the mating sequence between the male member and the female member when the BAR Carrier Assembly is being placed in the closed position shown in FIG. **31** according to the present invention;
FIG. **32** is a perspective view of the distal end of the shaft illustrating the BAR Carrier Assembly in the released position according to the present invention;
FIG. **33** is a cross-section view of the distal end of the shaft and BAR Carrier Assembly shown in FIG. **32** along lines **J-J** according to the present invention;
FIG. **34** is a perspective view of the protective showing the preferred embodiment according to the present invention;
FIG. **35** is a perspective view showing an alternative embodiment of the protective cover according to the present invention.
FIG. **36** is a perspective view showing an alternative embodiment of the collet according to the present invention;
FIG. **37** is a side view of the collet shown in FIG. **36** according to the present invention;
FIG. **38** is a perspective view showing an alternative embodiment of the drawbar according to the present invention;
FIG. **39** is an end view of the drawbar shown in FIG. **38** according to the present invention;
FIG. **40** is a cross-section view of the drawbar shown in FIG. **38** along lines **K-K** according to the present invention.

### DETAILED DESCRIPTION OF THE PREFERRED EMBODIMENTS

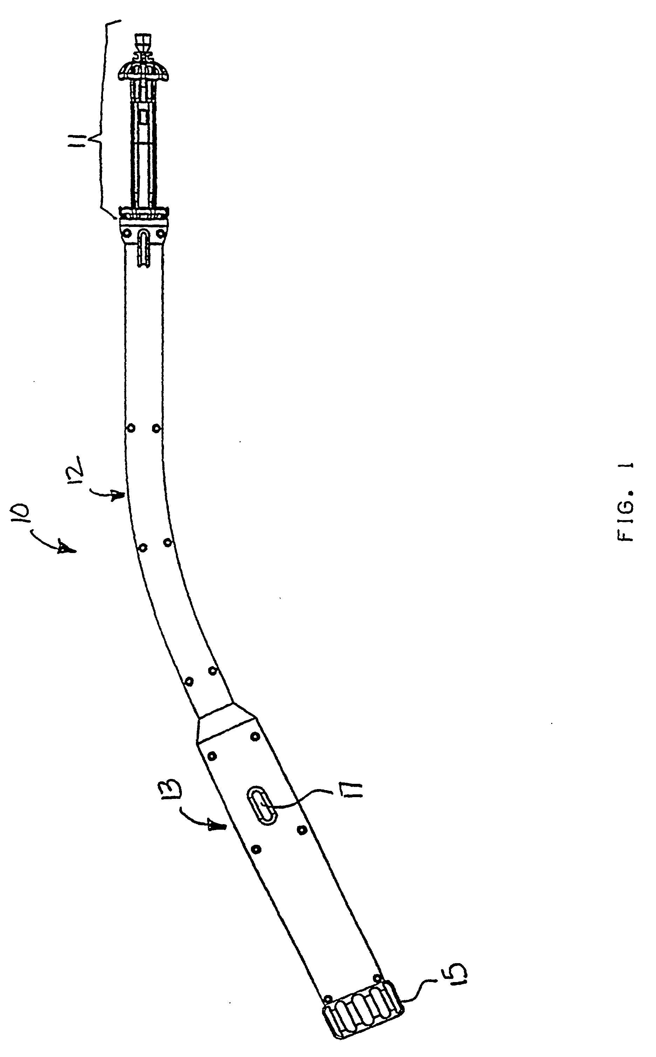

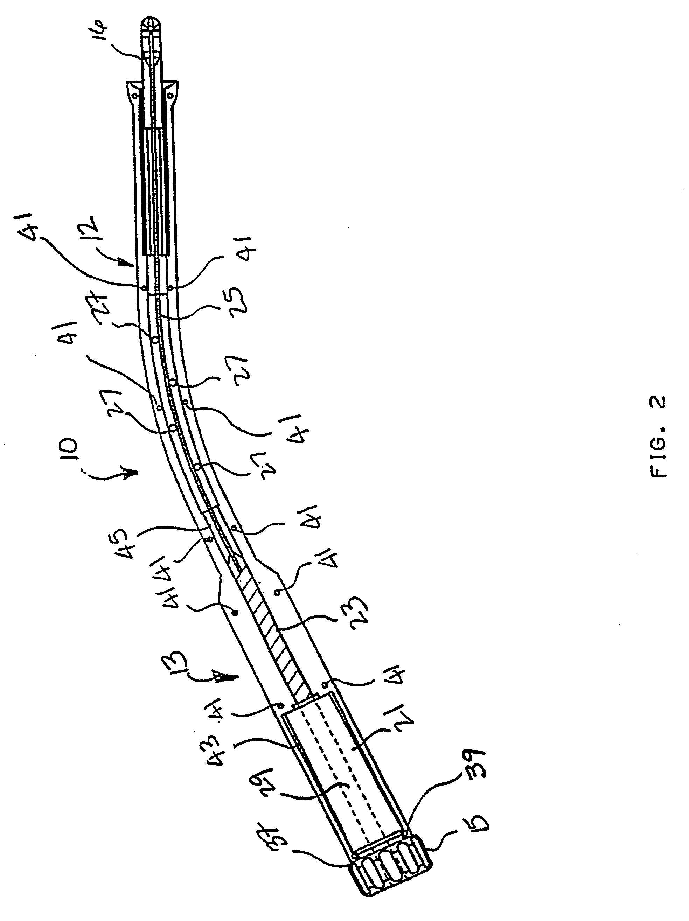

Referring to the drawings, the preferred embodiment of the anastomosis device of the present invention is illustrated and generally indicated as **10** in FIG. **1**. As shown in FIG. **1**, the anastomosis device **10** comprises a curved elongated shaft **12** integrally attached to or formed with a handle **13** at its proximal end, while a BAR carrier assembly **11** is attachable to distal end of shaft **12**. Referring to FIG. **2** a partial cross-section showing the interior of the anastomosis device **10** is illustrated. Handle **13** forms a hollow tubular configuration having connected first and second passageways **43** and **45**, respectively, that extend axially therethrough from a first opening **31** formed at the proximal end of anastomosis device **10** through the shaft **12** where the second passageway **45** terminates at a second opening **33** formed at the distal end thereof. The first passageway **43** extends axially from the first opening through the handle **13** where it communicates with the smaller diameter second passageway **45** where the handle **13** meets the shaft **12**.

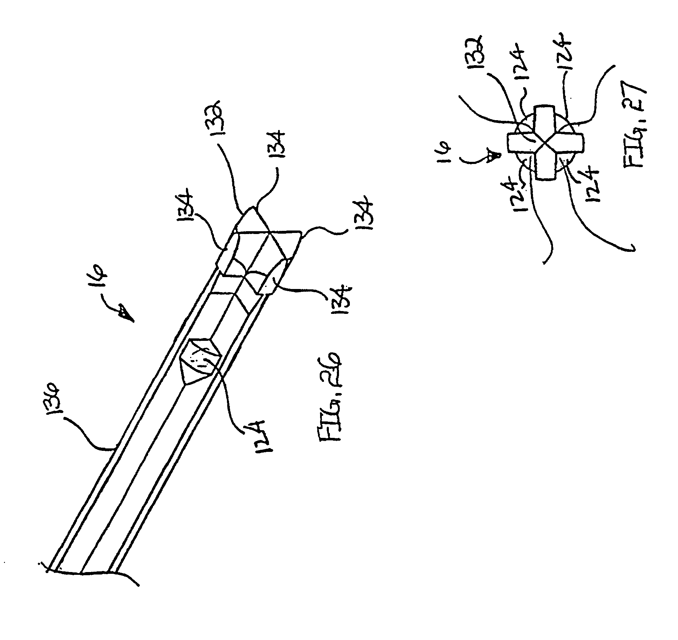

Preferably, shaft **12** comprises a two-part assembly that includes first and second identical halves **65** and **67** (FIG. **25**)that are attached together by pins (not shown)inserted and secured through holes **41** formed through the body of shaft **12**. Although the preferred embodiment discloses a pin and hole arrangement for securing first and second halves **65** and **67** together, it is within the scope of the present invention that halves **65** and **67** can be attached by other securing means to include, but not limited to, adhesive bonding, tongue and groove arrangement, riveting or other suitable means of attachment. Preferably, shaft **12** and handle **13** are made from a hard plastic material or any kind of material suitable for injection molding. However, in the alternative, metal may also be used to manufacture the shaft **12** and handle **13**.

A window **17** (FIG. **1**) is formed through handle **13** for providing a visual indication to the surgeon as to the state of closure of the BAR Carrier Assembly. A color mark or other visual indicator (not shown), such as a notch, is provided on a rod member **23** that is seen through window **17** when anastomosis device **10** is actuated and the BAR Carrier Assembly **11** is being placed in the closed position. The state of closure is determined by how much mark **10** fills window **17**, with full closure having mark **10** filling the entire window **17**.

A knob **15** having a gripping surface adapted for secure gripping between a user's thumb and forefinger is provided for actuating anastomosis device **10** from an open to a closed position, and finally to a release position. Knob **15** further includes an annular-shaped threaded body **21** with an undercut **39** interposed therebetween. The threaded body **21** is disposed in the first passageway **43** and forms an internal axial threaded channel **29** therethrough that includes a series of threads (not shown) adapted for engagement to rod member **23**. The distal end of first passageway **43** has a lip **37** adapted for engagement to undercut **39**, so that threaded body **21** is retained inside passageway **43** while permitting body **21** to freely rotate therein. Rod member 23 also has a series of threads formed around the outer surface thereof that mate and engage the threads of threaded body **21** when the proximal end of rod member **23** is inserted into the threaded channel **29** during assembly of the anastomosis device **10.** Rotation of knob **15** in a clockwise direction rotates threaded body **21** so that rod member **23** is pulled axially toward knob **15** while rotation of knob **15** in a counter-clockwise direction extends the rod member **23** away from knob **15**. Although the preferred embodiment of the present invention discloses a clockwise rotation of knob **15** for axially pulling in rod member **23** and a counter-clockwise rotation for axially extending away member **23**, it is within the scope of the present invention that rotation of knob **15** can be in either direction for pulling in or extending out member **23**.

Integrally attached to or formed with the distal end of rod member **23** is a flexible shaft member **25** that extends through the portion of the second passageway **45** that is formed through shaft **12**. A plurality of channeling members **27** are strategically located at points along the top and bottom portions of second passageway **45** for guiding the shaft member **25** along passageway **45** during its back-and-forth motion as the anastomosis device **10** is actuated, as shall be explained in greater detail later. The shaft member **25** is preferably a flat strip integrally attached to or formed with a cross-shaped drawbar **16** at the free end thereof. Drawbar **16** is adapted for engagement to the BAR carrier assembly **11** that is attachable to the distal end of anastomosis device **10**.

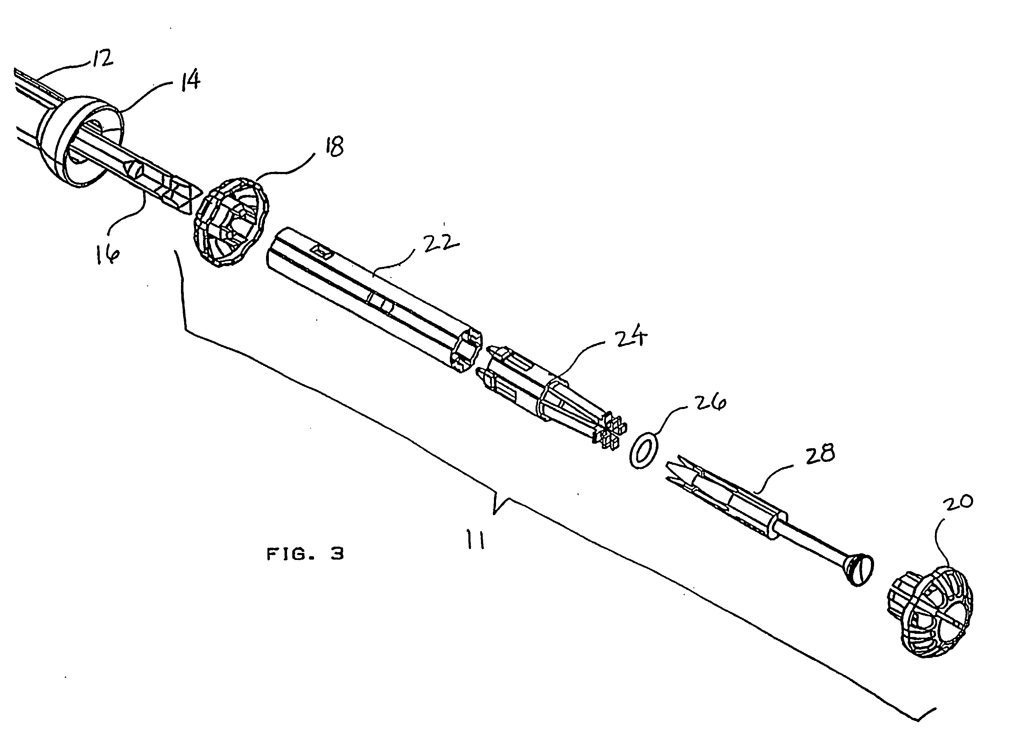

Referring to FIG. **3**, an exploded view of the distal end of the anastomosis device **10** and its attachable BAR carrier assembly **11** is shown. The BAR carrier assembly **11** comprises a BAR **19** that includes a female member **18** and a male member **20** that mate together to form a unitary biofragmentable anastomosis ring. As shall be discussed in greater detail later, female member **18** engages a female carrier **22** by inserting the carrier **22** through an aperture **54** formed through the female member **18**. Female carrier **22** has a hollow generally tubular shape with open ends and is adapted for engagement with shaft **12** when the proximal end of the carrier **22** is inserted into the second passageway **45**. A male carrier **24**, also forming a hollow generally tubular body with open ends and a plurality of legs **60** formed at the distal end thereof, is attachable to the female carrier **22** when the BAR Carrier Assembly **11** is placed in the open position. During manufacturing, a collet **28** is pre-loaded to the male carrier **24** and male member **20** and is provided for locking and subsequently releasing the wedge **84** of collet **28** from between the plurality of legs **60** of carrier **24** when collet **28** is inserted into the hollow generally tubular body of the male carrier **24** and through the plurality of legs **60**. Prior to locking wedge **84** between the plurality of legs **60**, legs **60** are inserted through the aperture **32** of male member **20** and engaged to the peripheral area around aperture **32**, so that when wedge **84** is inserted through legs **60** and biases apart legs **60**, the male member **20** is effectively locked to the male carrier **24**. A biasing means **26**, for example an O-ring or garter spring, is provided around the free end of the plurality of legs **60** for applying a counter-biasing force that locks against wedge **84** between the plurality of legs **60** and pre-loads the male member **20** to the male carrier **24**, as shall be explained in greater detail later.

Besides both male and female members **18** and **20**, BAR carrier assembly **11** further comprises respective female and male carriers **22** and **24** and collet **28** and is attachable to the distal end of shaft **12.** The distal end of shaft **12** forms an annular nest **14** where the second opening **33** communicates with the second passageway **45** formed inside shaft **12**. Nest **14** has a dome-like shape that forms a receptacle adapted for seating the dome-side of the female member **18**. Extending axially from the second opening **33** is drawbar **16**. As shall be explained in greater detail below, drawbar **16** is inserted through an aperture **54** formed in the center of the female member **18** that is seated in nest **14**, and is designed to engage and retain the BAR Carrier Assembly **11** to the shaft **12**.

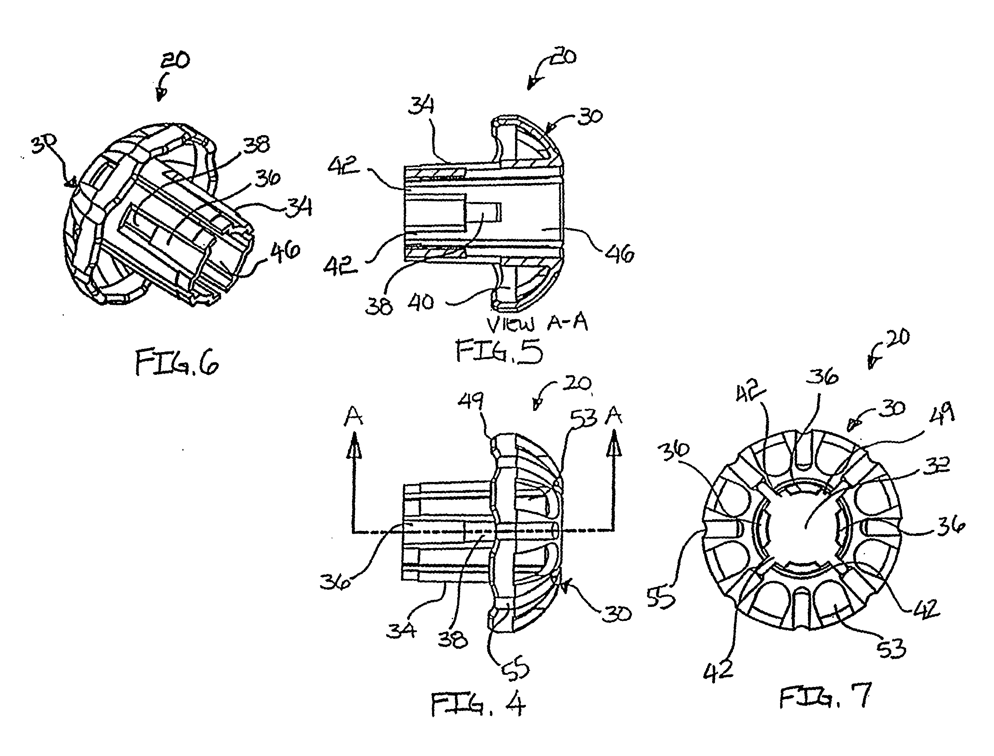

Referring to FIGS. **4-7**, the male member **20** is illustrated in greater detail. Male member **20** includes a dome **30** with an aperture **32** formed in center thereof, while a concentric pattern of openings **53** are in juxtaposition between scallops **55** formed around the exterior surface of dome **30.** Each opening **53** forms a bullet shaped aperture through dome **30** while each scallop **55** cuts a small arc along the outer edge **49** between each opening **53.** Dome **30** further includes an interior cavity **40** having an outer edge **49** which surrounds a tubular member **34** that extends axially from the aperture **54** through cavity **40**. A portion of the exterior surface of tubular member **34** forms a plurality of axially extending ring raceways **36** which are equally spaced around the circumference of member **34**. These ring raceways **36** are shallow channels extending axially along the exterior surface of tubular member **34** from the free end thereof to a point approximately halfway up member **34** where each raceway **36** terminates forming a slot **38** that communicates with the interior area of member **34**. The interior area of tubular member **34** forms a ring channel **46** that includes a series of axially extending grooves **42** spaced equally around the circumference of the interior surface thereof and formed between the protruding shallow channels of the ring raceways 36.

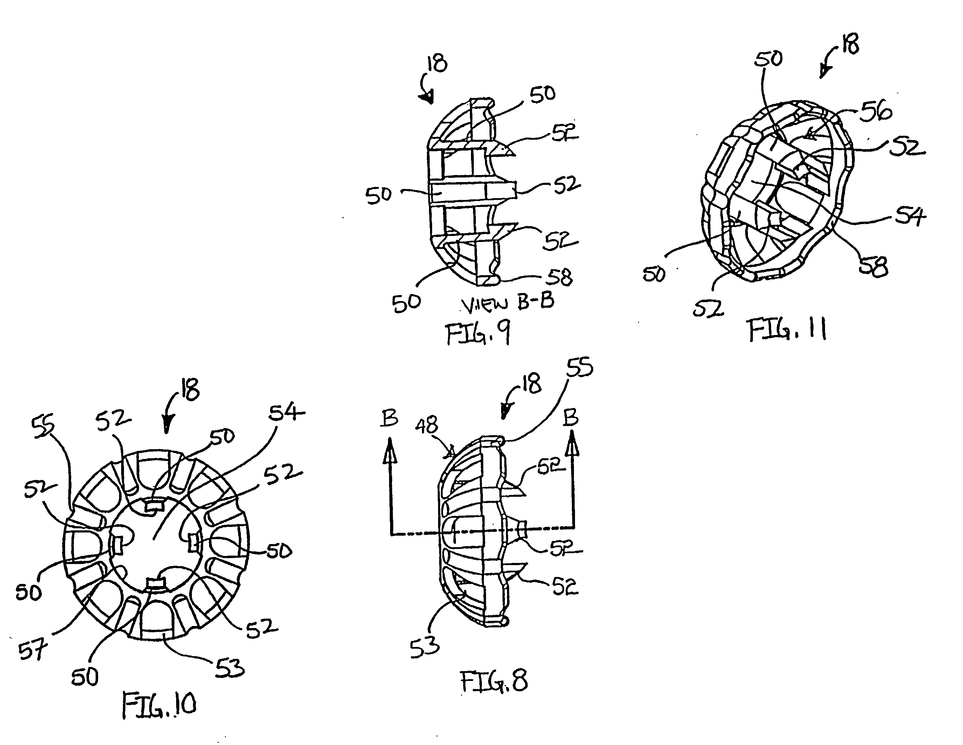

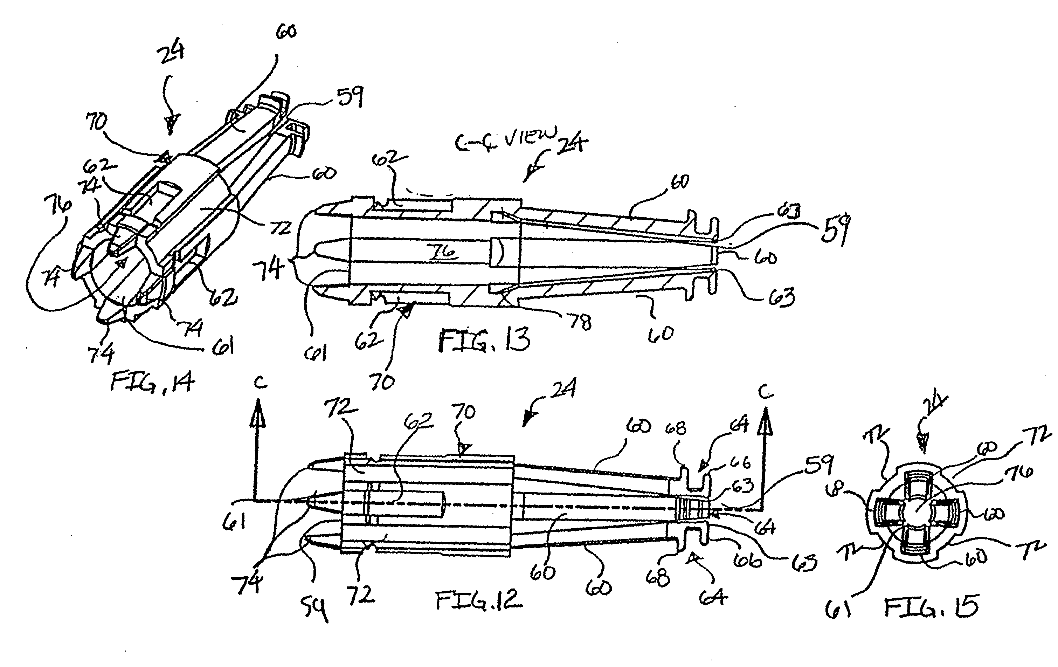

Referring to FIGS. **8-11**, the female member **18** is shown in greater detail. Female member **18** includes a concentric-shaped dome **48** similar configuration to dome **30** of male member **20** and has an interior cavity **56** having an outer edge **58** and an inner edge **57** that forms an axial aperture **54** in the center of dome **48**. Dome **48** further includes the same pattern of openings **53** and scallops **55** found on male member **20,** which form a similar concentric pattern as disclosed above. Extending axially from the inner edge **57** of aperture **54** and into the interior of cavity **56** are a plurality of mating prongs **50** with a pawl **52** formed at the free end of each prong **50**. Each pawl **52** forms a protruding portion adapted for engagement with a respective slot **36** of the male member **20** when the female member **18** is mated thereto. The leading edge of each pawl **52** is angled in order to provide a tunneling action that tunnels under and clears any obstruction, for example a severed bowel section, that might obstruct any one of the raceways **36** during mating of the male member **20** to the female member **18**.

Referring to FIGS. **12-15**, the male carrier **24** is shown in greater detail. Male carrier **24** comprises a carrier body **70** having a hollow generally tubular shape that forms a carrier channel **76** with opposing distal and proximal open ends **59** and **61**. The distal open end **59** forms the plurality of generally axially extending legs **60** that are spaced . equally around the circumference of open end **59** and set at a slight inward angle thereto. The free end of each leg **60** forms retaining jaws **64** having upper and lower jaws **66** and **68**, respectively. The upper and lower jaws **66** and **68** form a pocket adapted to receive a biasing means **26**, for example a resilient O-ring or garter spring, for biasing the plurality of legs **60** together, however any type of resilient biasing means is felt to fall within the scope of the present invention. Opposite each upper jaw **66** is a bump **63** adapted to engage and retain wedge **84** of collet **28**, as shall be explained in greater detail later. The carrier body **70** further includes a plurality of axially extending slots **62** spaced equally around the circumference of the proximal open end **61** in juxtaposition relation between each male carrier raceway **72**. Each male carrier raceway **72** forms an exterior shallow channel adapted for engaging and guiding the prongs **50** toward mating engagement with the male member **20** when the BAR Carrier Assembly is placed in the closed position. Provided at the proximal end of male carrier **24** are a plurality of connecting members **74** spaced equally around the circumference of the male carrier channel **76** at the entrance thereof and in axial alignment with the slots **62**. Connecting members **74** are adapted for engagement with connection slots **104** of female carrier **22** when male carrier **24** is connected thereto.

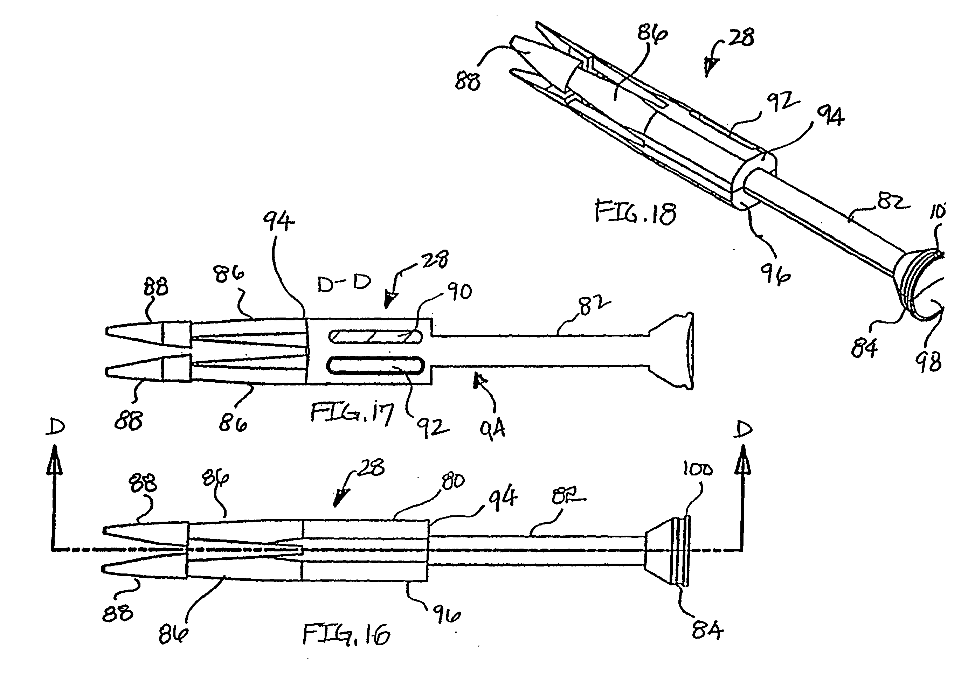

Referring to FIGS. **16-18**, collet **28** is shown in greater detail. Collet **28** serves as part of the release mechanism when the BAR **19** is being released from the BAR carrier assembly **11** and comprises first and second generally identical halves **94** and **96**, respectively, as illustrated in FIG. **18**. Preferably, the two halves **94** and **96** are attached to one another by tongue and groove arrangement strengthened by adhesive bonding, although any other suitable means of attachment, such as, but not limited to ultrasonic welding, riveting or a combination of any of the above, is felt, to fall within the scope of the present invention. Each half **94** and **96** includes opposing tongue **81** and **83** and groove **85** and **87** arrangements, respectively, for properly orienting and connecting the two halves **94** and **96** together by inserting the tongue **81** of half **94** into the groove **85** formed in half **96.** Conversely, the tongue **83** of half **96** is inserted into the groove **87** formed in half **94** when the two halves **94** and **96** are attached prior to bonding the two halves **94** and **96** together in a unitary structure using a suitable adhesive.

The proximal end of the collet body **80** includes a plurality of prongs **86** with a connector **88** formed at the free end of each respective prong **86**. The connectors **88** are adapted for engagement with the drawbar **16** while the distal end of the collet body **80** forms an elongated stem **82** having a wedge **84** at the free end thereof. The wedge **84** has a truncated conic section and a retaining ridge **100** formed around the upper section of wedge **84** adapted for retaining bump **63** of legs **60**. As shall be explained in greater detail later, the wedge **84** forms a part of the release mechanism that locks in as well as releases the BAR **19** during use of the anastomosis device **10**.

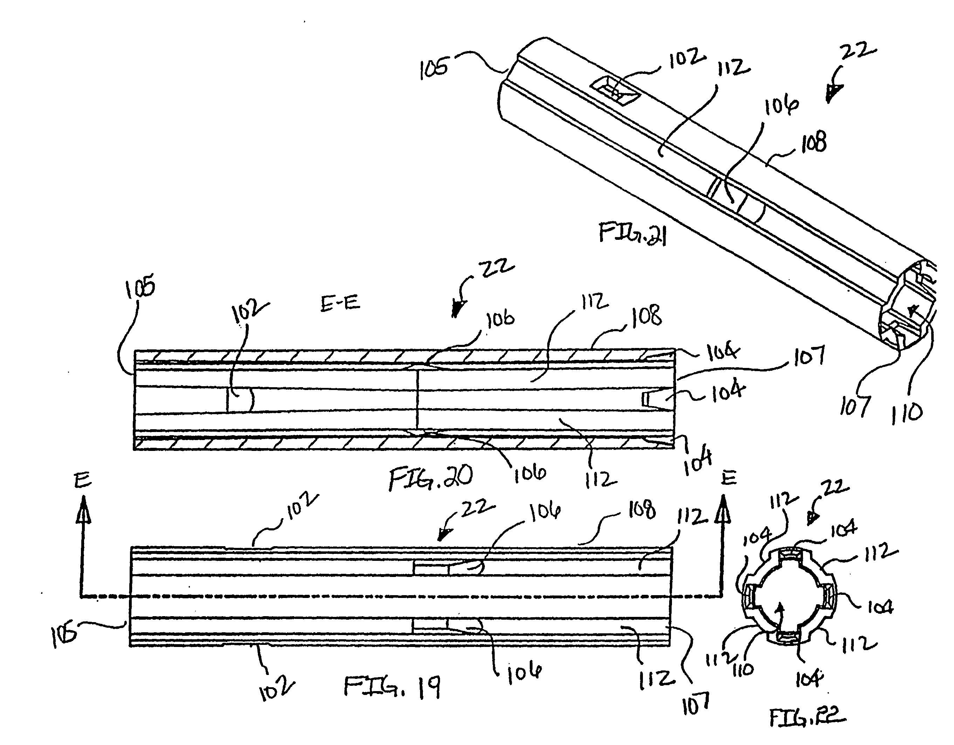

Referring to FIGS. **19-22**, the female carrier **22** is shown in greater detail. Female carrier **22** includes a carrier body **108** that has a generally hollow tubular shape with opposing proximal and distal open ends **105** and **107**. Formed through the carrier body **108** are opposing retaining windows **102** adapted for engagement with the shaft **12** when the female carrier **22** is inserted thereto. A means for guiding the female member **18** axially along the exterior surface of female carrier **22** is provided in the form of a pair of opposing female carrier raceways **112**. Female carrier raceways **112** comprise exterior shallow channels adapted for engaging and guiding the four mating prongs **50** of the female member **18** along the female carrier **22** until the prongs **50** engage retaining slots **106** formed at the mid point of each respective raceway **112**.

Distal open end **107** forms an annular opening that includes a plurality of connection slots **104** spaced equally around the circumference thereof, which are adapted for connection to a respective connecting member **74** of male carrier **24**. The connection slots **104** have beveled surfaces that facilitate engagement with the connection members **74** in a loose attachment fit thereto. Distal open end **107** forms one end of female carrier channel **110** that communicates through the hollow tubular configuration of female carrier **22** to the proximal opening **105**.

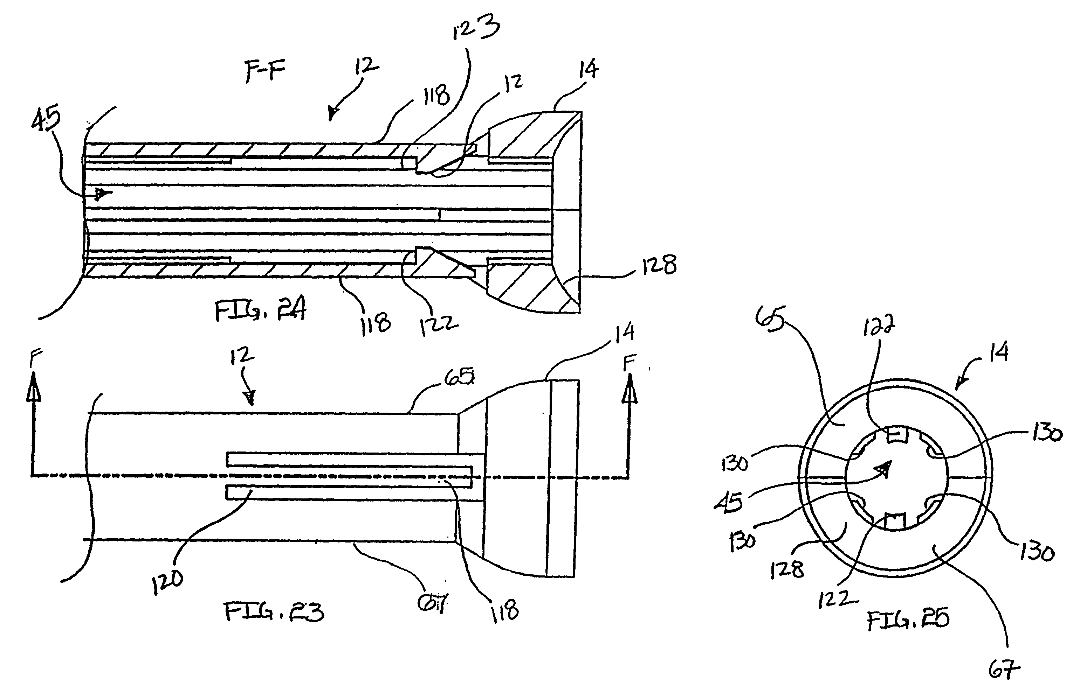

Referring to FIGS. **23-25**, the distal end of shaft **12** is shown in greater detail. Shaft **12** comprises two identical halves **65** and **67** that are preferably injection molded and attached to one another by adhesive bonding or other suitable means of attachment. As noted above, the nest **14** includes a flared cup-shaped receptacle **128** having the distal opening **89** formed in the center therethrough which communicates with second passageway **45**. Spaced apart from distal opening **89** of shaft **12** along the surface thereof are opposed shaft windows **120** that each form respective opposing retaining fingers **118**. Each retaining finger **118** includes a tang **122** at the free end thereof. Each tang **122** has a protruding portion **123** adapted to engage a respective retaining window **102** when the proximal open end **105** of female carrier **22** is inserted into the second passageway **45** through nest **14**. As illustrated in FIGS. **24** and **25**, second passageway **45** forms an axial channel with a plurality of guides **130** equally spaced around the circumference thereof for guiding the proximal open end **105** of the female carrier **22** into the passageway **45**. The guides **130** are adapted to engage the respective female carrier raceways **112** of female carrier **22** so that the carrier **22** slides into the second passageway **45** until the tangs **122** of the retention fingers **118** engage the retaining window **102** of female carrier **22**, thereby retaining the proximal open end **105** of the female carrier **22** inside shaft **12** while the distal open end **107** thereof extends out from passageway **45**.

As the female carrier **22** is inserted into second passageway **45**, the drawbar **16** disposed therein is concurrently inserted through the female carrier channel **110** of female carrier **22.** Drawbar **16** forms a cross-bar configuration having first, second, third and fourth axial channels **148, 150, 152** and **154** that extend the length of drawbar **16**. In order to engage the connectors **88** of collet **28**, the free end of drawbar **16** includes a cross bar **132** having four wings **134** with beveled edges adapted to engage the connectors **88** and prevent disengagement therefrom when drawbar **16** is pulled into second passageway **45**, as shall be discussed in greater detail below. Spaced apart from cross bar **132** along drawbar **16** and positioned in each channel **148, 150, 152** and **154** is a stop **124** provided for preventing the connectors **88** of collet **28** from being inserted too far down the drawbar **16** when the collet **28** is engaged thereto.

During manufacturing, collet **38** is pre-loaded to the male carrier **24** by inserting collet **28** through male carrier **24** so that the wedge **84** of collet **28** is placed between the plurality of legs **60** of carrier **24,** thereby biasing apart the legs **60**. Prior to inserting collet **28** through male carrier **24**, male member **20** is attached to the distal end of the male carrier **24**, so that each retaining jaw **64** formed at the free end of each leg **60** is advanced through aperture **32** formed in dome **30** of male member **20**. Once plurality of legs **60** are advanced through aperture **32** so that the retaining jaws **64** engage the peripheral area around the aperture **32**, the wedge **84** of collet **28** is then inserted through the carrier channel **76** of male carrier **24** until the retaining ridge **100** formed around the circumference of wedge **84** engages bump **63** formed opposite of each retaining jaw **64**, thereby biasing apart legs **60**. A biasing means **26**, for example a garter spring, is received inside each retaining jaw **64** so as to provide a counter force that locks the wedge **84** between the legs **60**, thereby locking the male member **20** to the distal open end **59** of male carrier **24.**

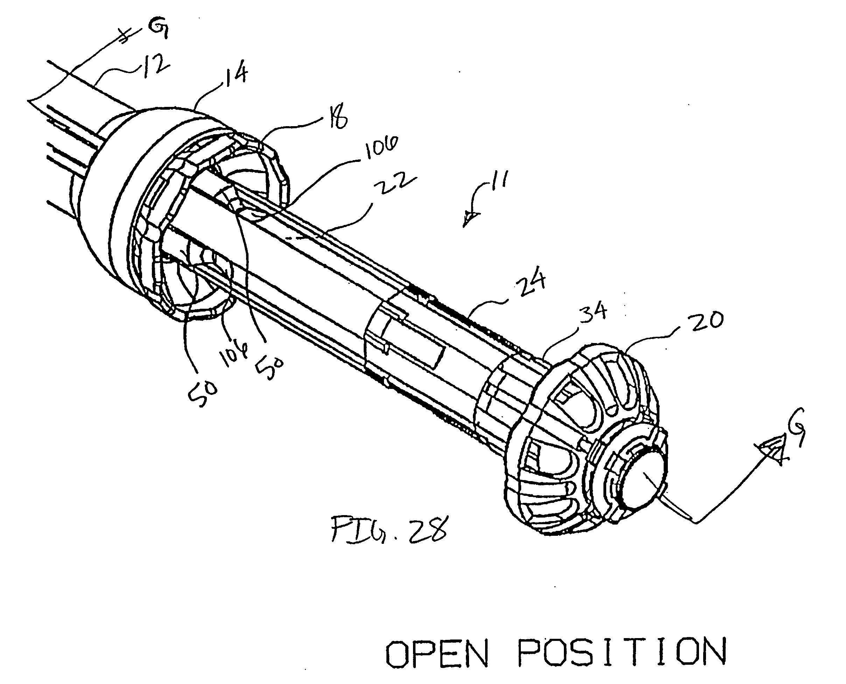

Referring to FIGS. **28-35,** the method for using the anastomosis device **10** will be discussed. During a surgical procedure for joining two severed halves of hollow body organs, such as intestines, the proximal open end **105** of female carrier **22** is inserted into the second passageway **45** of shaft **12** until the retaining fingers **118** engage the retaining windows **102** of shaft **12**, thereby pre-loading the carrier **22** to shaft **12**. Once the female carrier **22** is pre-loaded to the shaft **12**, carrier **22** is inserted through the aperture **54** of female member **18** until the dome **48** thereof is seated in nest **14.** Once the female member **18** is seated a protective cover **138** is inserted over the distal open end **107** of female carrier **22** in order to protect the patient during insertion of the anastomosis device **10** through the rectum.

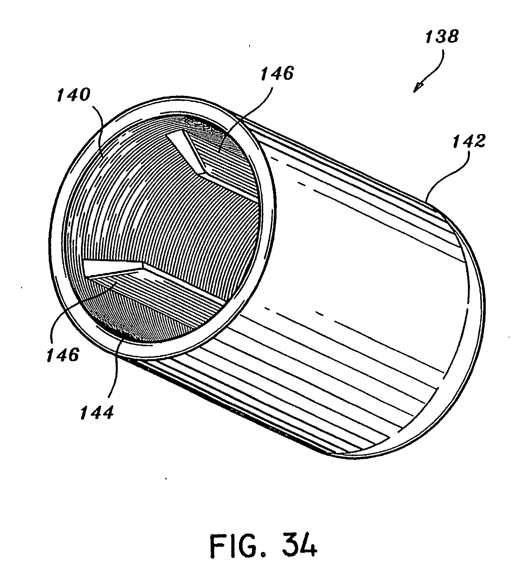

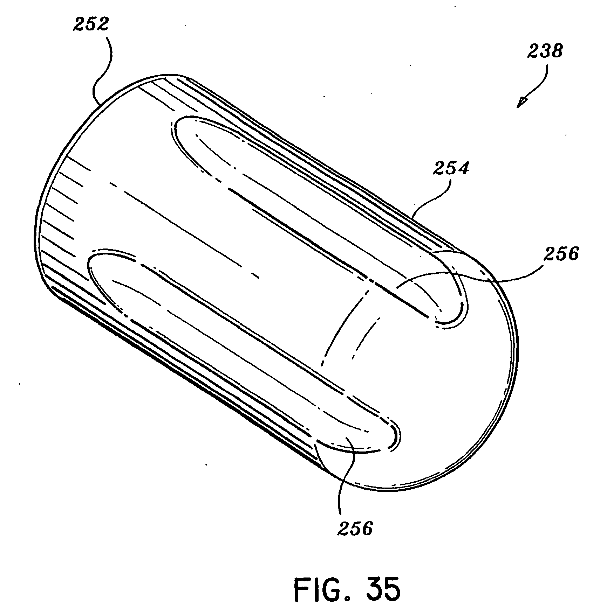

FIG. **34** shows the preferred embodiment of the protective cover **138**. Cover **138** comprises a cover body **140** having a bullet- shaped configuration that forms an interior cavity **140** with an opening **144** adapted to receive and retain the distal open end **107** of female carrier **22**. A plurality of axially extending retaining guides **146** are formed on the interior surface of cavity **140** for guiding the distal end of female carrier **22** and retaining carrier **22** therein. FIG. **35** shows an alternative embodiment of the protective cover **138**, designated **238**, is similar to the preferred embodiment discussed above except the cover **238** includes a cover body **254** that has a plurality of scallops **256** formed on the exterior surface thereof to assist the user in gripping the cover **238**.

In a surgical procedure to anastomosis two hollow body organs together, the surgeon first makes an abdominal opening by making an incision in the abdominal area of a patient in order to access the bowel region. The surgeon then severs the diseased portion of the bowel into a lower and an upper bowel sections (not shown), respectively, and cleans the respective edges of the severed bowel of any remaining diseased portions in order to ensure that only healthy tissue is to be anastomosed. Once the free ends of each bowel section have been cleaned, the surgeon inserts the pre-loaded male carrier **24** through the abdominal opening and secures the free end of the upper bowel section around the outer edge **49** of male member **20** in a contiguous manner using a purse string stitch that utilizes suitable absorbable sutures known in the art such as, but not limited to coated or uncoated polyglycolic acid suture. Once the upper bowel section has been secured, the surgeon inserts the distal end of shaft **12** of anastomosis device **10** into the rectum and through the bowel region until the distal end of shaft **12** appears through the lumen of the lower bowel section. The surgeon then secures the free end of the lower bowel section around the outer edge **55** of female member **18** using the same purse string stitch as described above.

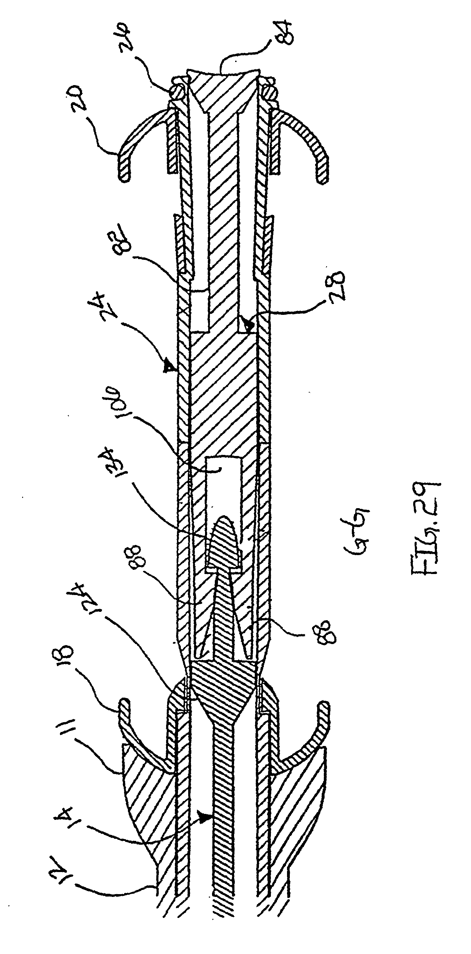

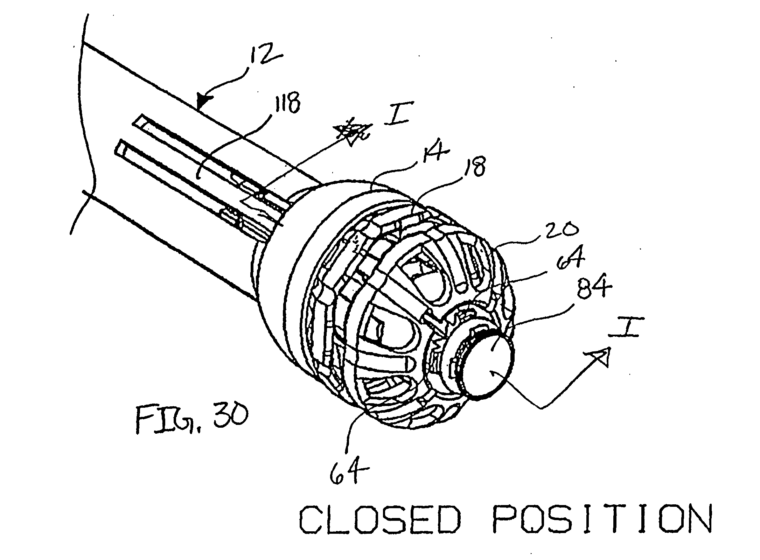

After the lower bowel section is properly secured to the female member **18,** the surgeon attaches the male carrier **24** to the female carrier 22 already attached to the drawbar **16** of shaft **12**. The attachment of the two carriers **22** and **24** is accomplished by engaging the connecting members **74** of the male carrier **24** to the connecting slots **104** of female carrier **22** in secure engagement thereto, thereby placing the BAR carrier assembly **11** in the open position, as illustrated in FIGS. **28** and **29**. Further, when male carrier **24** is attached to the female carrier **22,** the connectors **88** of collet **28** have already been attached to cross bar **132** of drawbar **16,** so that the collet **28** can release the male member **20** at the appropriate time when the device **10** is actuated and drawbar **16** is extended axially away from shaft **12** in order to release the female member **18** and male member **20** from the BAR Carrier Assembly **11**, as shall be discussed in greater detail below.

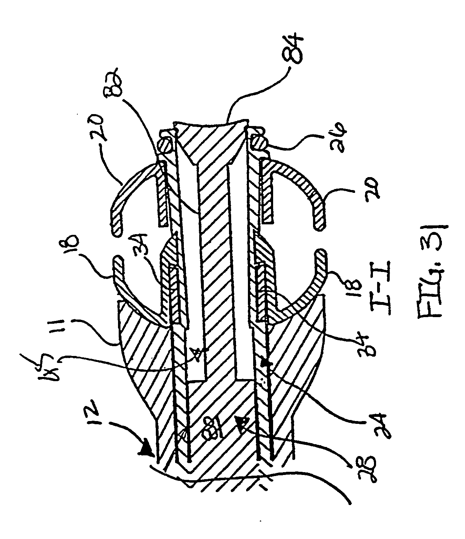

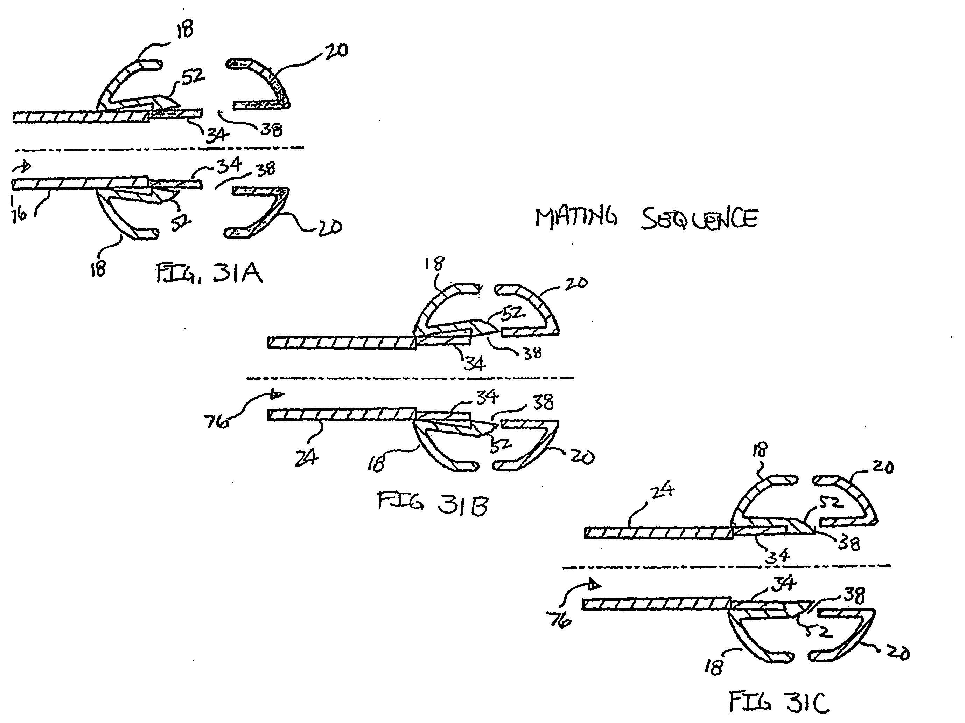

Referring to FIGS. **30** and **31,** the closed position of the BAR carrier assembly is shown. After the male member **20** is locked to the BAR carrier assembly **11** by collet **28** and is placed in the open position, the surgeon then actuates anastomosis device **10** so as to place the assembly **11** in the closed position and mate the male member **20** to the female member **18.** In order to place the BAR carrier assembly **11** in the closed position, the surgeon rotates the knob **15** in a clockwise direction, thereby rotating the rod member **23** in an axial direction toward knob **15**. This axial movement of the rod member **23** toward knob **15**, releases the female carrier **22** from engagement with retaining slots **106** of shaft **12**, so that the female carrier **22** and male carrier **24** are pulled into the second passageway **45** of the anastomosis device. This movement of the carriers **22** and **24** into the second passageway **45** concurrently causes the male member **20**, which is attached to the male carrier **24**, to move towards, and eventually mate with, the female member **18**.

Referring to FIG **31**, a cross-section view of the male member **20** fully mated to the female member **18** with the BAR carrier assembly **11** in the closed position is illustrated. As the male carrier **24** is pulled into the second passageway **45**, the pawl **52** of each mating prong **50** of female member **18** eventually engages the respective slot **38** formed in the tubular member **34** of the male member **20**, thereby mating the two members **18** and **20** in a unitary structure.

Referring to FIGS. **31A-31C**, the mating sequence between the female and male members **18** and **20** is illustrated. In FIG. **31A** the prongs **52** of female member **18** engage the ring raceway **36** of the tubular member **34** of male member **20** as the two members **18** and **20** begin to mate. FIG. **31B** illustrates the prongs **52** beginning to engage the slots **38** of male member **20** while FIG. **31C** shows the prongs **52** fully inserted into the slots **38** and the two unitary members **18** and **20** fully mated.

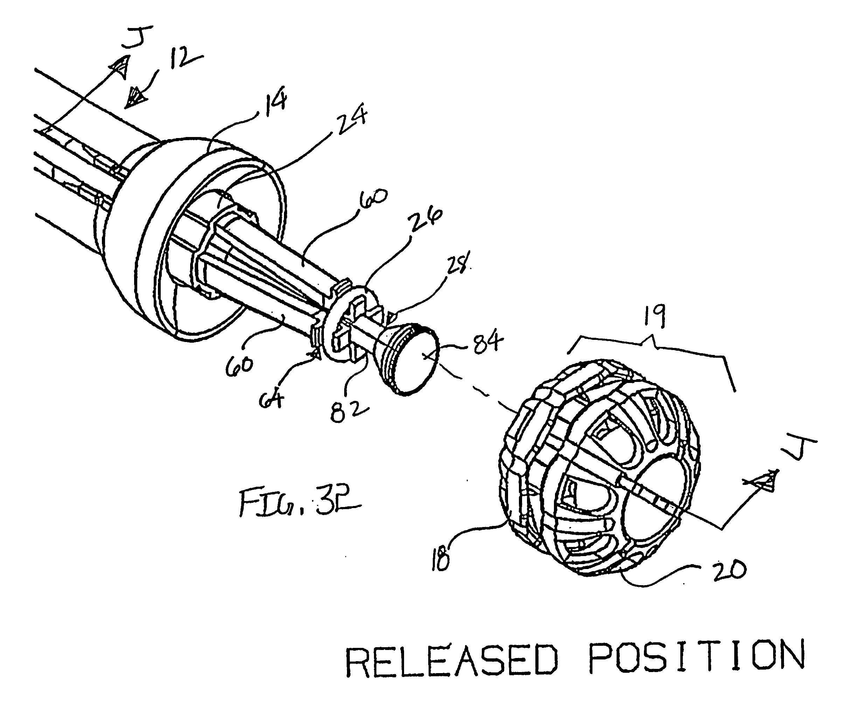

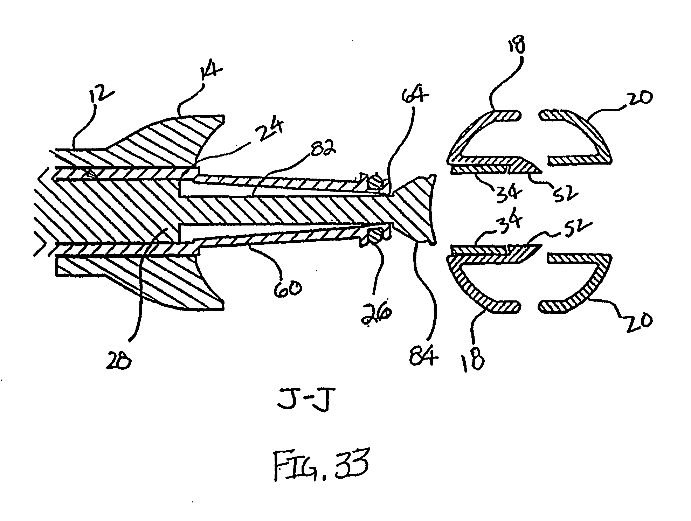

Referring to FIGS. **32** and **33**, the BAR carrier assembly **11** is shown in the release position. Once the unitary members **18** and **20** are mated, the surgeon rotates knob **15** in a counter-clockwise direction so that the drawbar **16** moves in an axial direction away from knob **15.** As the drawbar **16**, which is engaged to the prongs **86** of the collet **28,** moves axially, the wedge **84** is concurrently removed from between the legs **60** of male carrier **24,** thereby causing the legs **60** to collapse toward each other as the biasing means **26** forces the legs **60** inward and releases the BAR **19.**

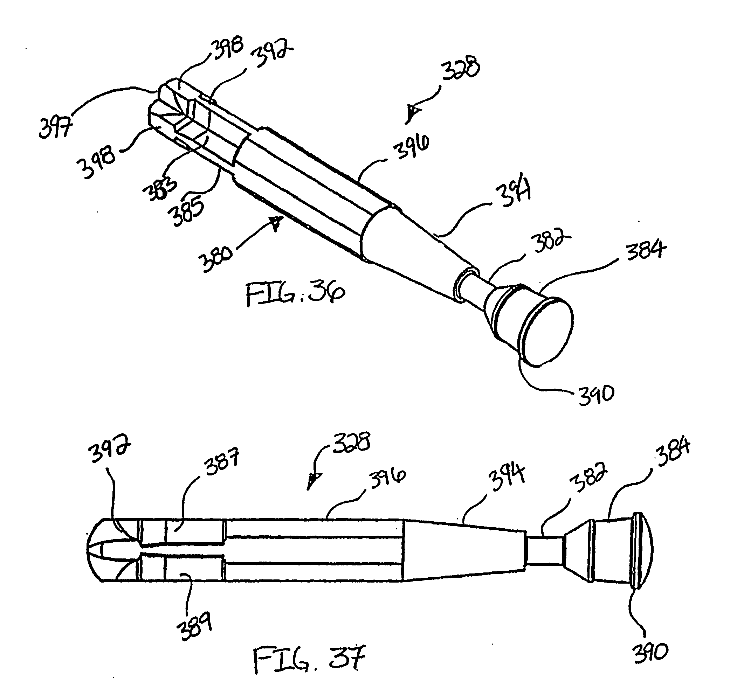

Referring to FIGS. **36** and **37,** an alternative embodiment of collet **28** is shown and generally designated as **328**. Collet **328** is of unitary manufacture and comprises a collet body **380** that includes a drawbar portion **392** formed at one end and a wedge **384** formed at the other end thereof. The drawbar portion **392** includes first, second, third and fourth axial channels **383, 385, 387** and **389** that extend axially along portion **392** and terminate at crossbar **397**. Cross bar **397** has a generally cross-bar configuration that forms first, second, third and fourth wings **370, 371, 372**, and **373,** respectively, at the free end of drawbar **316**. Each wing **370, 371, 372**, and **373** in combination with an adjacent wing **370, 371, 372**, or **373** forms an axially extending wing channel **399** that forms at the free end of drawbar portion **392** and tapers down to a respective channel **383, 385, 387** and **389**.

The collet body **380** further comprises a middle portion **396** of generally tubular cross section that is interposed between drawbar portion **392** and a front portion **394**. Front portion **394** has a conical member **395** that meets a stem **382** and terminates with a wedge **384** formed at the free end thereof. Wedge **384** is of similar construction as wedge **84** and is used in the similar lock and release arrangement described in the preferred embodiment.

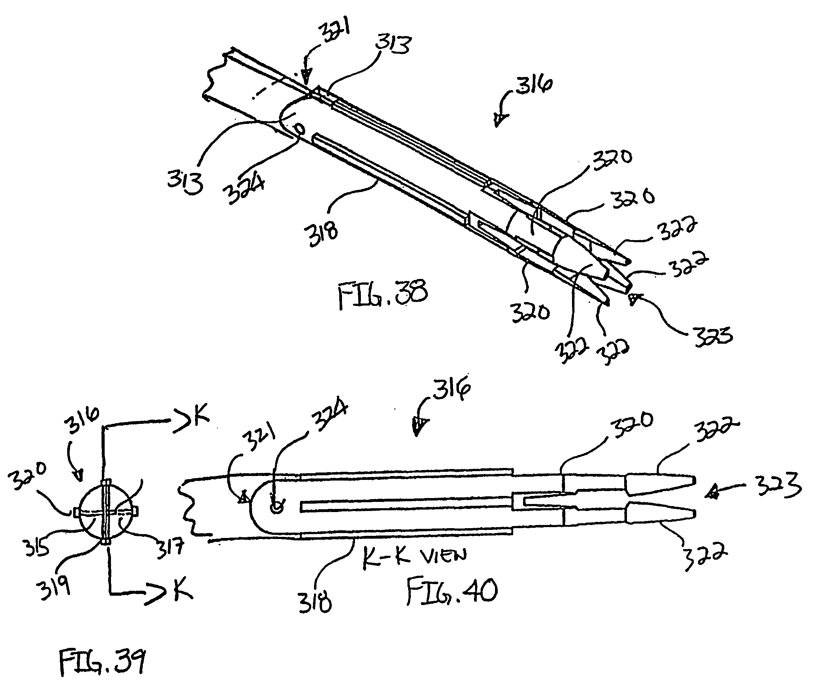

Referring to FIGS. **38-40**, an alternative embodiment of drawbar **16** is shown and generally designated as **316**. Drawbar **316** comprises to identical halves **315** and **317** which are attached to each other by adhesive bonding or other suitable means of attachment. Each wing **370, 371, 372**, and **373** of crossbar **397** is adapted for engagement with drawbar **316**. Drawbar **315** includes a proximal end **321** and a distal end **322.** As seen in FIG. **38**, proximal end **321** includes a pair rounded ends **313** with a slot **319** formed therebetween and a channel **324** that extends through the proximal end **321** in traverse relation to the axis of drawbar **316**. Slot **319** extends vertically between the pair of rounded edges **313** and is adapted to receive the free end of shaft member **25**. Preferably, shaft member **25** also includes an opening (not shown) that aligns with opening **324** when member **25** is attached to the proximal end **321** of drawbar **316.** A pin (not shown) is then inserted through the entire channel **324** and engaged thereto by means well known in the art so that shaft member **25** is secured to the drawbar **316**. Alternatively, other means of securing shaft member **25** to the proximal end **321** of drawbar **316** that fall within the scope of the present invention include, but are not limited to, a snap fit attachment, riveting or tongue and groove engagement.

Distal end **322** of drawbar **316** forms a plurality of axially extending connectors **320** that extend away from the free end thereof. Connectors **320** are of similar construction as the connectors **88** disclosed in the preferred embodiment of the collet **28** and are adapted for similar engagement with the drawbar portion **392** of collet **328** when the male carrier **24** is attached to the female carrier **22** by the surgeon when placing the BAR Carrier Assembly in the open position. The construction and structure of the other portions of the insertion device **10** remain unchanged.

It should be understood from the foregoing that, while particular embodiments of the invention have been illustrated and described, various modifications can be made thereto without departing from the scope of the invention. Therefore, it is not intended that the invention be limited by the specification; instead, the scope of the present invention is intended to be limited only by the appended claims.

## Claims

1. A medical device for use in joining of free ends of two tubular members comprising:
first (18) and second (20) ring members, said first and second ring members being securable to a respective free end of each tubular member to be anastomosed,
an insertion device (10) having a distal end and a proximal end, said insertion device adapted for transanally inserting the first of said ring members so that said first ring member extends through one of the free ends of the tubular members,
a carrier assembly (11) attachable to said distal end of said insertion device, said carrier assembly including first (22) and second (24) carriers for attachment and release of said first (18) and second (20) ring members, said first carrier being attachable to said distal end of said insertion device (10) and a distal end of said second carrier including a plurality of axially extending legs (60), and
said first (18) and second (20) ring members being positionable at first and second predetermined distances from each other;
**characterized by**:
said second carrier being attachable to said first carrier (22); and
further **characterized by**:
the distal ends of the legs being biased radially inward toward one another by application of a radial force to said distal ends thereof, said radial force being applied by a biasing means (26).

2. The medical device according to claim 1, wherein said first (18) and second (20) ring members each comprise a dome (30, 48), each of said domes further comprising an inner periphery (57) that forms an aperture (32, 54) and an outer periphery that forms an outer edge (49, 58).

3. The medical device according to claim 2, wherein each of said respective domes further forms an interior cavity (40, 56)

4. The medical device according to claim 1, wherein said first and second ring members are positionable in opposed relation to one another.

5. The medical device according to claim 3, wherein said first and second ring members are positionable in opposed relation to one another so that said interior cavity of each of said respective domes faces the interior cavity of the other.

6. The medical device according to claim 1, wherein when said first and second ring members are positioned at said first predetermined distance, said carrier assembly is in an open position.

7. The medical device according to claim 1, wherein when said first and second ring members are positioned at said second predetermined distance, said carrier assembly is in a closed position.

8. The medical device according to claim 1, wherein when said first and second ring members are positioned at said second predetermined distance, the respective secured free end of each of the tubular members are in contiguous contact with the other tubular member in an anastomotic state.

9. The medical device according to claim 7, wherein said second carrier (24) comprises a hollow tubular body forming a channel therethrough having distal and proximal open ends thereof, wherein said second carrier further comprises a plurality of axially extending second raceways (72) formed along an exterior of said second carrier, said second raceways adapted for guiding said first ring member (20) toward said second ring member (18) when said carrier assembly is placed in the closed position.

10. the medical device according to claim 9, wherein said first carrier (22) comprises a hollow tubular body forming a channel therethrough having distal and proximal open ends thereof, said first carrier further comprises a plurality of axially extending first raceways (112) formed along an exterior surface thereof, said first raceways adapted for guiding said first ring member (18) towards the second ring member when said carrier assembly is placed in the closed position.

11. The medical device according to claim 9, wherein each of said plurality of legs (60) has a pair of jaws (66, 68) at the free end thereof.

12. The medical device according to claim 11, wherein each said pair of jaws (66, 68) is adapted to receive said biasing means (26).

13. The medical device according to claim 12, wherein said biasing means (26) is a garter spring.

14. The medical device according to claim 12, wherein said biasing means (26) is an 0-ring.

15. The medical device according to claim 1, which further comprises a lock and release for locking said first ring member (18) to said first carrier (22) and subsequently releasing said first (18) and second (20) ring members from said carrier assembly (11) after said first and second ring members have been placed in said second predetermined distance from one another.

16. The medical device according to claim 15, wherein said lock and release comprises a collet body (28) having a proximal end and a distal end, said distal end of said collet body including an elongated stem (82) with a wedge (84) formed at the free end thereof.

17. The medical device according to claim 16, wherein said collet body is adapted for insertion through said proximal open end of said second carrier such that said wedge (84) biases apart said plurality of legs (60).

18. The medical device according to claim 17, wherein said second ring member (20) is locked in place to said distal open end of said second carrier (24) by placement of said plurality of legs (60) through said aperture of said first ring member and placement of said wedge (84) of said collet body between said plurality of legs, thereby forcing apart said plurality of legs by the presence of said wedge while said biasing means (26) simultaneously applies a counter force that locks said plurality of legs against said wedge.

19. The medical device according to claim 18, wherein said proximal end of said insertion member includes an actuator (15), said actuator being capable of effecting the release of said second ring member (20) from said second carrier (24) by moving said wedge (84) in an axial direction from between said plurality of legs such that said biasing means (26) collapses said plurality of legs (60) in the absence of said wedge and releases said second ring member.

20. The medical device according to claim 10, wherein when said first and second ring members are positioned at said first predetermined distance, said carrier assembly is placed in an open position.

21. The medical device according to claim 10, wherein when said first and second ring members are positioned at said second predetermined distance, said carrier assembly is placed in a closed position.

22. The medical device according to claim 21, wherein when said carrier assembly is placed in said closed position, said first ring member is mated to said second ring member in such a manner as to collectively form a sphere-like shape with a slot formed along the equator, said slot adapted for receiving thereabout said secured free ends of said tubular member in a contiguous manner.

23. The medical device according to claim 22, wherein said first ring member (18) is mated to said second ring member (20) by guiding said first ring member axially towards said second ring member along said first (112) and second (72) raceways.

24. The medical device according to claim 19, wherein said actuator includes a rotatable knob (15).

25. The medical device according to claim 2, wherein each of said domes (30, 48) includes a plurality of openings therethrough.

26. The medical device according to claim 2, wherein said inner periphery of each of said first and second members forms a bumpy pattern adapted for gripping the secured free ends of the tubular member therebetween.

27. The medical device according to claim 16, wherein said insertion device (10) comprises an actuator, said actuator being operably connected to a shaft member, said shaft member including a drawbar for operably engaging said proximal end of said collet body in order to release the mated first and second ring members.

28. The medical device according to claim 26, wherein said insertion device (10) further comprises a shaft (12) at said distal end thereof and a handle (13) formed at the proximal end thereof.

29. The medical device according to claim 28, wherein said handle forms a window (17), said window giving a visual indication of the state of the actuation of said carrier assembly.

30. The medical device according to claim 28, wherein said visual indication of the state of actuation is a color mark.

31. The medical device according to claim 28, wherein said visual indication of the state of actuation is a notch formed through said shaft member, said notch providing said visual indication of the state of actuation by the percentage of said notch that is visible through said window.

32. The medical device according to claim 10, wherein when said carrier assembly is placed in said closed postion, said actuator operates to axially pull said first carrier (22) into the distal end of said insertion device (10) until said second carrier (24) is received into said distal end, thereby mating said first and second ring members.

33. The medical device according to claim 23, wherein said distal end of said insertion device (10) forms a nest (14), said nest adapted to receive said first ring member (18).

34. The medical device according to claim 32, wherein to mate said first and second ring members, said first ring member (18) is disposed in said nest (14) and said second ring member (20) is driven axially towards said first ring member along said first (22) and second (24) carriers until said first ring member mates with said second ring member.

## Patentansprüche

1. Medizinische Vorrichtung zum Verbinden freier Enden von zwei rohrförmigen Elementen, umfassend:
ein erstes (18) und ein zweites (20) Ringelement, wobei das erste und das zweite Ringelement an einem jeweiligen freien Ende eines jeden, zu anastomosierenden rohrförmigen Elements anbringbar sind,
eine Einführvorrichtung (10) mit einem distalen Ende und einem proximalen Ende, wobei die Einführvorrichtung zum transanalen Einführen des ersten der Ringelemente ausgebildet ist, so dass sich das erste Ringelement durch eines der freien Enden der rohrförmigen Elemente erstreckt,
eine Trägereinheit (11), die an dem distalen Ende der Einführvorrichtung befestigbar ist, wobei die Trägereinheit einen ersten (22) und einen zweiten (24) Träger zum Befestigen und Freigeben des ersten (18) und des zweiten (20) Ringelements umfasst, und wobei der erste Träger an dem distalen Ende der Einführvorrichtung (10) befestigbar ist, und ein distales Ende des zweiten Trägers eine Mehrzahl von sich in axialer Richtung erstreckenden Beinen (60) umfasst, und
das erste (18) und das zweite (20) Ringelement mit einem ersten und einem zweiten vorbestimmten Abstand zueinander positionierbar sind,
**dadurch gekennzeichnet, dass**
der zweite Träger an dem ersten Träger (22) befestigbar ist, und dass die distalen Enden der Beine in radialer Richtung nach innen zueinander hin durch Ausüben einer Radialkraft auf die distalen Enden derselben vorgespannt sind, wobei die Radialkraft durch ein Vorspannmittel (26) ausgeübt wird.

2. Medizinische Vorrichtung nach Anspruch 1, wobei sowohl das erste (18) als auch das zweite (20) Ringelement eine Rundung (30, 48) aufweist, und jede der Rundungen ferner einen Innenumfang (57), der eine Öffnung (32, 54) bildet, und einen Außenumfang, der eine Außenkante (49, 58) bildet, aufweist.

3. Medizinische Vorrichtung nach Anspruch 2, wobei jede der Rundungen ferner einen Innenhohlraum (40, 56) bildet.

4. Medizinische Vorrichtung nach Anspruch 1, wobei das erste und das zweite Ringelement gegenüberliegend zueinander positionierbar sind.

5. Medizinische Vorrichtung nach Anspruch 3, wobei das erste und das zweite Ringelement gegenüberliegend zueinander derart positionierbar sind, so dass der Innenhohlraum einer Rundung zum Innenhohlraum der anderen Rundung gerichtet ist.

6. Medizinische Vorrichtung nach Anspruch 1, wobei die Trägereinheit sich in einer offenen Position befindet, wenn das erste und das zweite Ringelement um den ersten vorbestimmten Abstand zueinander positioniert sind.

7. Medizinische Vorrichtung nach Anspruch 1, wobei die Trägereinheit in einer geschlossenen Position ist, wenn das erste und das zweite Ringelement um den zweiten vorbestimmten Abstand zueinander positioniert sind.

8. Medizinische Vorrichtung nach Anspruch 1, wobei das jeweilige angebrachte freie Ende eines jeden rohrförmigen Elements sich in angrenzendem Kontakt mit dem anderen rohrförmigen Element in einem anastomosischen Zustand befindet, wenn das erste und das zweite Ringelement um den zweiten vorbestimmten Abstand zueinander positioniert sind.

9. Medizinische Vorrichtung nach Anspruch 7, wobei der zweite Träger (24) einen hohlförmigen, rohrförmigen Korpus aufweist, der einen Kanal hierdurch mit distalen und proximalen offenen Enden bildet, und wobei der zweite Träger des weiteren eine Mehrzahl von sich in axialer Richtung erstreckenden zweiten Laufflächen (72) aufweist, die entlang einer Außenseite des zweiten Trägers gebildet sind, und wobei die zweiten Laufflächen zum Führen des ersten Ringelements (20) zum zweiten Ringelement (18) hin ausgebildet sind, wenn die Trägereinheit in der geschlossenen Position angeordnet ist.

10. Medizinische Vorrichtung nach Anspruch 9, wobei der erste Träger (22) einen hohlförmigen, rohrförmigen Korpus aufweist, der einen Kanal hierdurch mit distalen und proximalen offenen Enden bildet, und wobei der erste Träger des weiteren eine Mehrzahl von sich in axialer Richtung erstreckenden ersten Laufflächen (112) aufweist, die entlang einer Außenoberfläche desselben gebildet sind, und wobei die ersten Laufflächen zum Führen des ersten Ringelements (18) zum zweiten Ringelement hin ausgebildet sind, wenn die Trägereinheit in der geschlossenen Position angeordnet ist.

11. Medizinische Vorrichtung nach Anspruch 9, wobei jedes Bein (60) ein Paar Backen (66, 68) an dem freien Ende desselben besitzt.

12. Medizinische Vorrichtung nach Anspruch 11, wobei jedes Backenpaar (66, 68) zur Aufnahme des Vorspannmittels (26) ausgebildet ist.

13. Medizinische Vorrichtung nach Anspruch 12, wobei das Vorspannmittel (26) eine Ringbandfeder ist.

14. Medizinische Vorrichtung nach Anspruch 12, wobei das Vorspannmittel (26) ein O-Ring ist.

15. Medizinische Vorrichtung nach Anspruch 1, ferner umfassend eine Ver- und Entriegelung zum Verriegeln des ersten Ringelements (18) mit dem ersten Träger (22) und anschließendem Entriegeln des ersten (18) und des zweiten (20) Ringelements von der Trägereinheit (11), nachdem das erste und das zweite Ringelement um den zweiten vorbestimmten Abstand zueinander angeordnet worden sind.

16. Medizinische Vorrichtung nach Anspruch 15, wobei die Ver- und Entriegelung einen Spannzangenkörper (28) mit einem proximalen Ende und einem distalen Ende aufweist, wobei das distale Ende des Spannzangenkörpers einen länglichen Schaft (82) mit einem an dessen freien Ende gebildeten Keil (84) umfasst.

17. Medizinische Vorrichtung nach Anspruch 16, wobei der Spannzangenkörper zum Einführen durch das proximale offene Ende des zweiten Trägers ausgebildet ist, so dass der Keil (84) die Mehrzahl der Beine (60) auseinander bewegt.

18. Medizinische Vorrichtung nach Anspruch 17, wobei das zweite Ringelement (20) mit dem distalen offenen Ende des zweiten Träger (24) dadurch verriegelt ist, indem die Mehrzahl der Beine (6.0) durch die Öffnung des ersten Ringelements geführt und der Keil (84) des Spannzangenkörpers zwischen die Mehrzahl der Beine gebracht wird, wodurch die Mehrzahl der Beine durch den Keil auseinander bewegt werden, während das Vorspannmittel (26) gleichzeitig eine Gegenkraft ausübt, die die Mehrzahl der Beine mit dem Keil verriegelt.

19. Medizinische Vorrichtung nach Anspruch 18, wobei das proximale Ende des Einführelements ein Betätigungselement (15) umfasst, und wobei das Betätigungselement in der Lage ist, die Entriegelung des zweiten Ringelements (20) von dem zweiten Träger (24) auszulösen, indem der Keil (84) in axialer Richtung aus einer Position zwischen der Mehrzahl der Beine bewegt wird, so dass das Vorspannmittel (26) die Mehrzahl der Beine (60) in Abwesenheit des Keils zusammen bewegt und das zweite Ringelement entriegelt.

20. Medizinische Vorrichtung nach Anspruch 10, wobei die Trägereinheit in einer offenen Position angeordnet ist, wenn das erste und das zweite Ringelement um den ersten vorbestimmten Abstand positioniert sind.

21. Medizinische Vorrichtung nach Anspruch 10, wobei die Trägereinheit in einer geschlossenen Position angeordnet ist, wenn das erste und das zweite Ringelement um den zweiten vorbestimmten Abstand positioniert sind.

22. Medizinische Vorrichtung nach Anspruch 21, wobei das erste Ringelement mit dem zweiten Ringelement derart zusammengeführt wird, dass sie beide gemeinsam eine kugelförmige Form mit einem entlang dem Äquator gebildeten Schlitz bilden, wenn die Trägereinheit in der geschlossenen Position angeordnet ist, wobei der Schlitz zum Aufnehmen der angebrachten freien Enden des rohrförmigen Elements etwa dort auf angrenzende Weise ausgebildet ist.

23. Medizinische Vorrichtung nach Anspruch 22, wobei das erste Ringelement (18) mit dem zweiten Ringelement (20) zusammengeführt wird, indem das erste Ringelement in axialer Richtung zum zweiten Ringelement hin entlang der ersten (112) und der zweiten (72) Lauffläche geführt wird.

24. Medizinische Vorrichtung nach Anspruch 19, wobei das Betätigungselement einen Drehknopf (15) umfasst.

25. Medizinische Vorrichtung nach Anspruch 2, wobei jede der Rundungen (30, 48) eine Mehrzahl von hierdurch verlaufenden Öffnungen umfasst.

26. Medizinische Vorrichtung nach Anspruch 2, wobei der Innenumfang des ersten und des zweiten Elements ein unebenes Muster bildet, das zum Greifen der angebrachten freien Enden des rohrförmigen Elements dazwischen ausgebildet ist.

27. Medizinische Vorrichtung nach Anspruch 16, wobei die Einführvorrichtung (10) ein Betätigungselement aufweist, und wobei das Betätigungselement betriebsmäßig mit einem Schaftelement verbunden ist, und das Schaftelement eine Deichsel zum betriebsmäßigen Ineingriffbringen des proximalen Endes des Spannzangenkörpers umfasst, um die zusammengeführten ersten und zweiten Ringelemente zu entriegeln.

28. Medizinische Vorrichtung nach Anspruch 26, wobei die Einführvorrichtung (10) ferner einen Schaft (12) an dem distalen Ende desselben und einen Griff (13), der an dem proximalen Ende desselben gebildet ist, aufweist.

29. Medizinische Vorrichtung nach Anspruch 28, wobei der Griff ein Fenster (17) bildet, und wobei das Fenster eine visuelle Anzeige des Betätigungszustands der Trägereinheit gibt.

30. Medizinische Vorrichtung nach Anspruch 28, wobei die visuelle Anzeige des Betätigungszustands eine Farbmarkierung ist.

31. Medizinische Vorrichtung nach Anspruch 28, wobei die visuelle Anzeige des Betätigungszustands eine durch das Schaftelement gebildete Kerbe ist, und wobei die Kerbe eine visuelle Anzeige des Betätigungszustands über den Anteil der Kerbe, der durch das Fenster sichtbar ist, vorsieht.

32. Medizinische Vorrichtung nach Anspruch 10, wobei, wenn die Trägereinheit in der geschlossenen Position angeordnet ist, das Betätigungselement den ersten Träger (22) in axialer Richtung in das distale Ende der Einführvorrichtung (10) so weit zieht, bis der zweite Träger (24) in dem distalen Ende aufgenommen ist, wodurch das erste und das zweite Ringelement zusammengeführt werden.

33. Medizinische Vorrichtung nach Anspruch 23, wobei das distale Ende der Einführvorrichtung (10) ein Nest (14) bildet, und wobei das Nest zur Aufnahme des ersten Ringelements (18) ausgebildet ist.

34. Medizinische Vorrichtung nach Anspruch 32, wobei zum Zusammenführen des ersten und des zweiten Ringelements das erste Ringelement (18) in dem Nest (14) angeordnet ist, und das zweite Ringelement (20) in axialer Richtung zum ersten Ringelement hin entlang dem ersten (22) und dem zweiten (24) Träger bewegt wird, bis das erste Ringelement mit dem zweiten Ringelement zusammengeführt ist.

## Revendications

1. Dispositif médical utilisé pour relier des extrémités libres de deux éléments tubulaires comprenant :
des premier (18) et second (20) éléments annulaires, lesdits premier et second éléments annulaires pouvant être fixés à une extrémité libre respective de chaque élément tubulaire à anastomoser,
un dispositif d'insertion (10) présentant une extrémité distale et une extrémité proximale, ledit dispositif d'insertion étant conçu pour insérer d'une manière transanale le premier desdits éléments annulaires de telle sorte que ledit premier élément annulaire s'étend à travers l'une des extrémités libres des éléments tubulaires,
un ensemble porteur (11) pouvant être fixé à ladite extrémité distale dudit dispositif d'insertion, ledit ensemble porteur comprenant des premier (22) et second (24) porteurs pour la fixation et la libération desdits premier (18) et second (20) éléments annulaires, ledit premier porteur pouvant être fixé à ladite extrémité distale dudit dispositif d'insertion (10), et une extrémité distale dudit second porteur incluant plusieurs branches s'étendant axialement (60), et
lesdits premier (18) et second (20) éléments annulaires pouvant être positionnés à des première et seconde distances prédéterminées l'un de l'autre ;
**caractérisé par**
ledit second porteur pouvant être fixé audit premier porteur (22) ; et
**caractérisé en outre par** :
les extrémités distales des branches étant sollicitées radialement vers l'intérieur, l'une vers l'autre, par l'application d'une force radiale auxdites extrémités distales de celles-ci, ladite force radiale étant appliquée par un moyen de sollicitation (26).

2. Dispositif médical selon la revendication 1, où lesdits premier (18) et second (20) éléments annulaires comprennent chacun un dôme (30, 48), chacun desdits dômes comprenant en outre une périphérie interne (57) qui forme une ouverture (32, 54) et une périphérie externe qui forme un bord extérieur (49, 58).

3. Dispositif médical selon la revendication 2, où chacun desdits dômes respectifs forme en outre une cavité intérieure (40, 56).

4. Dispositif médical selon la revendication 1, où lesdits premier et second éléments annulaires peuvent être positionnés en une relation opposée l'un à l'autre.

5. Dispositif médical selon la revendication 3, où lesdits premier et second éléments annulaires peuvent être positionnés en une relation opposée l'un à l'autre de telle sorte que ladite cavité intérieure de chacun desdits dômes respectifs est orientée vers la cavité intérieure de l'autre.

6. Dispositif médical selon la revendication 1, où lorsque lesdits premier et second éléments annulaires sont positionnés à ladite première distance prédéterminée, ledit ensemble porteur se trouve dans une position ouverte.

7. Dispositif médical selon la revendication 1, où lorsque lesdits premier et second éléments annulaires sont positionnés à ladite seconde distance prédéterminée, ledit ensemble porteur se trouve dans une position fermée.

8. Dispositif médical selon la revendication 1, où lorsque lesdits premier et second éléments annulaires sont positionnés à ladite seconde distance prédéterminée, l'extrémité libre respective fixée de chacun des éléments tubulaires est en contact contigu avec l'autre élément tubulaire dans un état d'anastomose.

9. Dispositif médical selon la revendication 7, où ledit second porteur (24) comprend un corps tubulaire creux formant un canal à travers celui-ci présentant des extrémités distale et proximale ouvertes de celui-ci, où ledit second porteur comprend en outre plusieurs seconds chemins (72) s'étendant axialement formés le long d'un extérieur dudit second porteur, lesdits seconds chemins étant aptes à guider ledit premier élément annulaire (20) vers ledit second élément annulaire (18) lorsque ledit ensemble porteur est placé dans la position fermée.

10. Dispositif médical selon la revendication 9, où ledit premier porteur (22) comprend un corps tubulaire creux formant un canal à travers celui-ci ayant des extrémités distale et proximale ouvertes de celui-ci, ledit premier porteur comprend en outre plusieurs premiers chemins (112) s'étendant axialement formés le long de sa surface extérieure, lesdits premiers chemins étant aptes à guider ledit premier élément annulaire (18) vers le second élément annulaire lorsque ledit ensemble porteur est placé dans la position fermée.

11. Dispositif médical selon la revendication 9, où chacune de la pluralité de branches (60) présente une paire de mâchoires (66, 68) à son extrémité libre.

12. Dispositif médical selon la revendication 11, où chacune desdites paires de mâchoires (66, 68) est apte à recevoir ledit moyen de sollicitation (26).

13. Dispositif médical selon la revendication 12, où ledit moyen de sollicitation (26) est un ressort cylindrique en anneau.

14. Dispositif médical selon la revendication 12, où ledit moyen de sollicitation (26) est un joint torique.

15. Dispositif médical selon la revendication 1, qui comprend en outre un verrouillage et un relâchement pour verrouiller ledit premier élément annulaire (18) audit premier porteur (22) et pour relâcher ensuite lesdits premier (18) et second (20) éléments annulaires dudit ensemble porteur (11) après que lesdits premier et second éléments annulaires ont été placés dans ladite seconde distance prédéterminée l'un de l'autre.

16. Dispositif médical selon la revendication 15, où ledit verrouillage et relâchement comprend un corps de collet (28) avec une extrémité proximale et une extrémité distale, ladite extrémité distale dudit corps de collet comprenant une tige oblongue (82) avec un coin (84) formé à son extrémité libre.

17. Dispositif médical selon la revendication 16, où ledit corps de collet est conçu pour l'insertion à travers ladite extrémité proximale ouverte dudit second porteur de telle sorte que ledit coin (84) sollicite ladite pluralité de branches (60) les unes au loin des autres.

18. Dispositif médical selon la revendication 17, où ledit second élément annulaire (20) est verrouillé en place à ladite extrémité distale ouverte dudit second porteur (24) par un placement de ladite pluralité de branches (60) à travers ladite ouverture dudit premier élément annulaire et un placement dudit coin (84) dudit corps de collet entre ladite pluralité de branches, en écartant ainsi de force ladite pluralité de branches par la présence dudit coin pendant que ledit élément de sollicitation (26) applique simultanément une contre-force qui verrouille ladite pluralité de branches contre ledit coin.

19. Dispositif médical selon la revendication 18, où ladite extrémité proximale dudit élément d'insertion comprend un actionneur (15), ledit actionneur étant apte à effectuer le relâchement dudit second élément annulaire (20) dudit second porteur (24) en déplaçant ledit coin (84) dans une direction axiale depuis entre ladite pluralité de branches de telle sorte que ledit moyen de sollicitation (26) entraîne l'affaissement de ladite pluralité de branches (60) en l'absence dudit coin et libère ledit second élément annulaire.

20. Dispositif médical selon la revendication 10, où lorsque lesdits premier et second éléments annulaires sont positionnés à ladite première distance prédéterminée, ledit ensemble porteur est placé dans une position ouverte.

21. Dispositif médical selon la revendication 10, où lorsque lesdits premier et second éléments annulaires sont positionnés à ladite seconde distance prédéterminée, ledit ensemble porteur est placé dans une position fermée.

22. Dispositif médical selon la revendication 21, où lorsque ledit ensemble porteur est placé dans ladite position fermée, ledit premier élément annulaire est amené à correspondre audit second élément annulaire de manière à former collectivement une forme de sphère avec une fente ménagée le long de l'équateur, ladite fente étant destinée à recevoir autour de celle-ci lesdites extrémités libres fixées dudit élément tubulaire d'une manière contiguë.

23. Dispositif médical selon la revendication 22, où ledit premier élément annulaire (18) est apparié audit second élément annulaire (20) en guidant ledit premier élément annulaire axialement vers ledit second élément annulaire le long desdits premier (112) et second (72) chemins.

24. Dispositif médical selon la revendication 19, où ledit actionneur comprend un bouton tournant (15).

25. Dispositif médical selon la revendication 2, où chacun desdits dômes (30, 48) présente plusieurs ouvertures à travers celui-ci.

26. Dispositif médical selon la revendication 2, où ladite périphérie interne de chacun desdits premier et second éléments forment un motif à bosses apte à saisir les extrémités libres fixées de l'élément tubulaire entre elles.

27. Dispositif médical selon la revendication 16, où ledit dispositif d'insertion (10) comprend un actionneur, ledit actionneur étant relié fonctionnellement à un élément d'arbre, ledit élément d'arbre comprenant un tirant pour venir fonctionnellement en prise avec ladite extrémité proximale dudit corps de collet pour libérer les premier et second éléments annulaires assemblés.

28. Dispositif médical selon la revendication 26, où ledit dispositif d'insertion (10) comprend en outre un arbre (12) à son extrémité distale précitée et une poignée (13) formée à son extrémité proximale.

29. Dispositif médical selon la revendication 28, où ladite poignée forme une fenêtre (17), ladite fenêtre fournissant une indication visuelle de l'état d'actionnement dudit ensemble porteur.

30. Dispositif médical selon la revendication 28, où ladite indication visuelle de l'état d'actionnement est une marque de couleur.

31. Dispositif médical selon la revendication 28, où ladite indication visuelle de l'état d'actionnement est une encoche formée à travers ledit élément d'arbre, ladite encoche fournissant ladite indication visuelle de l'état d'actionnement par le pourcentage de ladite encoche qui est visible à travers ladite fenêtre.

32. Dispositif médical selon la revendication 10, où lorsque ledit ensemble porteur est placé dans ladite position fermée, ledit actionneur fonctionne pour tirer axialement ledit premier porteur (22) dans l'extrémité distale dudit dispositif d'insertion (10) jusqu'à ce que ledit second porteur (24) soit reçu dans ladite extrémité distale, en unissant ainsi les premier et second éléments annulaires.

33. Dispositif médical selon la revendication 23, où ladite extrémité distale dudit dispositif d'insertion (10) forme un nid (14), ledit nid étant apte à recevoir ledit premier élément annulaire (18).

34. Dispositif médical selon la revendication 32, où pour unir lesdits premier et second éléments annulaires, ledit premier élément annulaire (18) est disposé dans ledit nid (14), et ledit second élément annulaire (20) est entraîné axialement vers ledit premier élément annulaire le long desdits premier (22) et second (24) porteurs jusqu'à ce que ledit premier élément annulaire s'unisse avec ledit second élément annulaire.
